(19) Europäisches Patentamt
European Patent Office
Office européen des brevets

(11) **EP 3 546 438 A1**

(12) **EUROPEAN PATENT APPLICATION**

(43) Date of publication:
**02.10.2019 Bulletin 2019/40**

(51) Int Cl.:
***C07C 5/333*** (2006.01)   ***C07C 15/46*** (2006.01)
***B01J 21/04*** (2006.01)   ***B01J 21/18*** (2006.01)
***B01J 37/02*** (2006.01)   ***B01J 37/08*** (2006.01)

(21) Application number: **18305336.2**

(22) Date of filing: **26.03.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Université de Strasbourg**
  **67000 Strasbourg (FR)**
• **Centre National de la Recherche Scientifique**
  **75016 Paris (FR)**
• **Consiglio Nazionale Delle Ricerche**
  **00185 Roma (IT)**
• **Ecole Centrale De Lille**
  **59650 Villeneuve d'Ascq (FR)**
• **Ecole Nationale Superieure De Chimie De Lille**
  **59652 Villeneuve D'Ascq (FR)**
• **Universite D'Artois**
  **62000 Arras (FR)**
• **Université des Sciences et Technologies de Lille 1**
  **59650 Villeneuve-d'Ascq (FR)**

(72) Inventors:
• **BA, Housseinou**
  **67200 STRASBOURG (FR)**
• **GIAMBASTIANI, Giuliano**
  **50041 FIRENZE (IT)**
• **TUCI, Giulia**
  **51018 PIEVE A NIEVOLE (PISTOIA) (IT)**
• **NGUYEN-DINH, Lam**
  **DA-NANG (VN)**
• **GRANGER, Pascal**
  **59260 HELLEMMES-LILLE (FR)**
• **PHAM-HUU, Cuong**
  **67100 STRASBOURG (FR)**

(74) Representative: **Novagraaf Technologies
Bâtiment O2
2, rue Sarah Bernhardt
CS90017
92665 Asnières-sur-Seine Cedex (FR)**

(54) **PROCESS FOR THE NON-OXIDATIVE DEHYDROGENATION OF A HYDROCARBON AND USES THEREOF**

(57)     The present invention relates to a process for the direct dehydrogenation of a hydrocarbon, comprising the step of contacting said hydrocarbon with a catalyst at a temperature comprised between 400°C and 700 °C, wherein said catalyst consists of a carbon active phase that is coated on γ-Al2O3 support, said γ-Al2O3 support being a host matrix for said carbon active phase.

It also relates to a process for the preparation of a composite comprising a carbon active phase coating on a γ-Al2O3 support, said γ-Al2O3 support being a host matrix for said carbon active phase, said process comprising the step of treating a support consisting of γ-Al2O3 at a temperature comprised of from 550°C to 650°, under an ethane stream for a period of at least 1.5 hours or under a stream of ethylbenzene, ethane or ethylbenzene diluted in an inert gas stream.

Other subject-matters of the invention are a composite comprising a support consisting of γ-Al2O3 and a carbon active phase, wherein said γ-Al2O3 support is a host matrix for said carbon active phase, obtainable by a process as defined above, and the use of the composite, for oxygen- and/or steam-free direct dehydrogenation of ethylbenzene to styrene.

EP 3 546 438 A1

Figure 1

**Description**

**Technical field**

The present invention refers to a process for the direct dehydrogenation of a hydrocarbon, comprising the step of contacting said hydrocarbon with a catalyst, and a process of preparation of a composite comprising the catalyst.
Therefore, the present invention has utility in the field of chemical industry, and especially petrochemical industry.
In the description below, references into brackets **([])** refer to the list of references located at the end of the text.

**Background of the Invention**

Styrene (ST) is industrially produced by the direct dehydrogenation of ethylbenzene (EB) over an iron oxide-based heterogeneous catalyst stabilized with potassium and alumina, in the presence of an excess of steam (water vapor) at reaction temperatures ranging from 580 to 630 °C (R. A. Meyer. Handbook of Petrochemicals Production processes. New York NY, McGraw-Hill, 11.3-11.34 (2005) **([1])**). The excess of steam used in the process plays a dual role: (i) it reduces the excessive formation of coke at the surface of the catalyst during the reaction, said coke inducing a progressive deactivation of the catalyst itself, and (ii) it reduces the temperature gap in the catalytic bed for this endothermic process. The process as such is a high-energy demanding transformation with relatively high costs due to the generation of the necessary amount of steam, and additional costs for the treatment of wastewater leaving the reactor. To solve this problem, researchers have focused their efforts in the search of robust catalysts, capable of operating with the same catalytic performances, but in the absence of steam. Results have shown that carbon can fulfill this role. Carbon-based catalysts, as metal-free systems, have been studied by various research groups worldwide and have been used in two key dehydrogenation processes, i.e. the production of styrene (ST) from ethylbenzene (EB). These two processes are namely (i) the oxidative dehydrogenation (ODH) in the presence of oxygen and (ii) the direct dehydrogenation (DDH).
The ODH process is an exothermic transformation and it generally takes place under milder conditions compare to DDH because of the presence of oxygen; therefore, ODH promoted by metal-free catalysts is generally more studied than DDH from the various research teams.
Regarding carbon-based catalysts as metal-free systems for the oxidative dehydrogenation (ODH), recent works have shown that the carbon phase formed at the surface of selected supports (i.e. alumina or silica-modified alumina) during the dehydrogenation reaction in the presence of oxygen, exhibits catalytic performances for the EB conversion to ST (T. G. Alkhazov, A. E. Lisovskii. Role of condensation products in the oxidative dehydrogenation process of ethylbenzene on alumina-oxide catalyst. Kinet. Catal. 17, 375-379, 1976 **([2])**; G. E. Vrieland, P. G. Menon. Nature of the catalytically active carbonaceous sites for the oxydehydrogenation of ethylbenzene to styrene - A brief review. Appl. Catal. 77, 1-8, 1991 **([3])**; C. Nederlof, F. Kapteijn, M. Makkee. Catalysed ethylbenzene dehydrogenation in CO2 or N2 - Carbon deposits as the active phase. Appl. Catal. A: Gen. 417, 163-173, 2012 **([4])**; N. Keller, N. I. Maksimova, V. V. Roddatis, M. Schur, G. Mestl, Y. V. Butenko, V. L. Kuznetsov, R. Schlogl. The catalytic use of onion-like carbon materials for styrene synthesis by oxidative dehydrogenation of ethylbenzene. Angew. Chem. Int. Ed. 41, 1885-1888, 2002 **([5])**; M. F. R. Pereira, J. L. Figueiredo, J. J. M. Orfao, P. Serp, P. Kalck, Y. Kihn. Catalytic activity of carbon nanotubes in the oxidative dehydrogenation of ethylbenzene. Carbon 42, 2807-2813, 2004 **([6])**). Authors agree that alumina itself has no dehydrogenating activity and that the catalytically active phase consists in the carbon deposit formed during the reaction at the surface of the inorganic oxide (Vrieland and Menon ([3]); R. Fiedorow, W. Przystajko, M. Sopa, I. G. Dalla Lana. The nature and catalytic influence of coke formed on alumina: oxidative dehydrogenation of ethylbenzene. J. Catal. 68, 33-41, 1981 **([7])**; V. Zarubina, C. Nederlof, B. Van der Linden, F. Kapteijn, H. J. Heeres, M. Makkee, I. Melian-Cabrera. Making coke a more efficient catalyst in the oxidative dehydrogenation of ethylbenzene using wide-pore transitional alumina. J. Mol. Catal. A: Chem. 381, 179-187, 2014 **([11])**; T. Wang, S. Chong, T. Wang, H. Lu, M. Ji. The physicochemical properties and catalytic performance of carbon-covered alumina for oxidative dehydrogenation of ethylbenzene with CO2. Appl. Surf. Sci. 427, 1011-1018, 2018 **([12])**). Nevertheless, the ODH process lists some technical drawbacks and limitations such as: 1) the relatively low selectivity in ST ($S_{ST}$ <95%); 2) the difficult control of an accurate concentration of the reagents in the stream and 3) the potentially dangerous use of oxygen in the stream which may cause serious hazards for the plant installation. It is worthy to note that all *in-situ* prepared carbon-based catalysts reported so far, have been used as metal-free catalysts for the alkane dehydrogenation process only under oxidative conditions (ODH), whereas no tests for the direct dehydrogenation (DDH) of alkanes has ever been carried out with these systems up to date.
Regarding carbon-based catalysts for the direct alkane dehydrogenation (DDH), the paper by Su et al. (J. Zhang, D. S. Su. R. Blume, R. Schlögl, R. Wang, X. Yang, A. Gajovic. Surface Chemistry and Catalytic Reactivity of a nanodiamond in the Steam-Free dehydrogenation of Ethylbenzene. Angew. Chem. Int. Ed. 49, 8640-8644, 2010 **([8])**) has demonstrated first that C-nanomaterials (i.e nanodiamonds (ND) generated by detonation) can be used as effective catalysts for the EB dehydrogenation in the absence of steam. Ba et al. have recently reported on the use of nanodiamonds supported on various materials such as alumina, silicon carbide, carbon nanofibers, as a catalyst for the process (H. Ba, L. Truong-Phuoc, Y. Liu, C. Duong-Viet, J. M. Nhut, L. Nguyen-Dinh, P. Granger, C. Pham-Huu. Hierarchical carbon nanofibers/graphene composite containing nanodiamonds as metal-free catalyst for direct dehydrogenation of ethylbenzene. Carbon 96, 1060-1069, 2016 **([9])** ; H. Ba, Y. Liu, X. Mu, J. M. Nhut, W. H. Doh, P. Granger, C. Pham-Huu. Nanodiamonds decorated silicon carbide foam as metal-free catalyst for steam-free ethylbenzene dehydrogenation. Appl. Catal. A: Gen. 499, 217-226, 2015 **([10])**, Y. Liu, H. Ba, J. Luo, J. M. Nhut, D. S. Su, C. Pham-Huu. Structure-performance relationship of nanodiamonds@nitrogen-doped mesoporous carbon in the direct dehydrogenation of ethylbenzene. Catal. Today 301, 38-47, 2018 **([13])**). Notably, dehydrogenation activity and catalyst stability (in the function of time) of ND-based catalysts supported on hierarchically structured materials, increase significantly. However, production costs of ND along with costs of hierarchical supports make these composites rather unattractive as catalysts despite their interesting performance. In addition, limitations related to handling and transportation of materials with nanoscopic dimensions such as ND as well as the severe conditions typically required for their large scale production are brakes that limit an effective industrial exploitation of this type of materials as catalysts. Finally yet importantly, the progressive deactivation of the catalyst active phase due to the progressive generation of coke deposits under the dehydrogenation conditions, render these systems undesirable from an industrial viewpoint.

Therefore, the development of alternative catalytic materials and processes for a highly selective and effective oxygen- and steam-free dehydrogenation catalysis of hydrocarbons, especially ethylbenzene (EB), is a priority for making easier the process implementation at industrial level and reducing costs.

**Description of the invention**

The inventors have developed a new and straightforward approach to the preparation of metal-free catalysts for the highly selective and effective oxygen- and steam-free dehydrogenation catalysis of hydrocarbons. Their catalysts consist of an active carbon layer coating generated at the surface of an alumina support (namely, gamma alumina - $\gamma$-$Al_2O_3$).

The $\gamma$-$Al_2O_3$ as support is commercially available and it is produced through a well-implemented industrial process that does not require post-synthetic treatments - a net advantage compared to other commercially available supports. Such an organic-inorganic composite has been employed as a metal-free system for an industrially relevant catalytic (heterogeneous) process at the heart of polymer industry. In particular, results have shown how this system is a highly selective and efficient catalyst for the direct dehydrogenation (DDH) process of hydrocarbons, especially in the conversion of EB to ST, under steam-free conditions. The composite displays a very high direct dehydrogenation performance in terms of ethylbenzene conversion along with extremely high styrene selectivity, equal to 90 %, preferentially higher than 95% and especially higher than 98% under industrial reaction conditions.

One of the numerous advantages of the catalyst of the invention is that the carbon-based active phase (alumina coating) can be synthesized using either *in-situ* or *ex-situ* methods. Under industrial-like operation conditions and under steam-free conditions, the composite of the invention displays a high stability as a function of time-on-stream thus indicating that catalyst deactivation by both surfaces fouling with carbonaceous residues or leaching is unlikely to occur.

The main advantages of the composite of the invention are (i) a carbon-based metal-free catalyst with low cost and easy scale-up production, (ii) a highly active and selective direct dehydrogenation catalyst operating under steam-free conditions, (iii) a stable catalyst for long-term operation under industrial-like conditions, and (iv) a direct and easy "shaping" of macroscopic industrial supports i.e. $\gamma$-$Al_2O_3$, with controlled macroscopic shapes. The carbon layer deposited on the $\gamma$-$Al_2O_3$ support provides the catalyst with a higher thermal conductivity which can reduce the problem of temperature gradient during the operating process, especially under steam-free conditions.

As mentioned above, the catalyst of the invention may be produced with a macroscopic shape, for example in the form of pellets, rings or foams, usually encountered within industrial plants, thus allowing to reduce as much as possible the problem of replacement.

In a first aspect, the present invention refers to a process for the direct dehydrogenation of a hydrocarbon, comprising the step of contacting said hydrocarbon with a catalyst at a temperature comprised between 400°C and 700 °C, wherein said catalyst consists of a carbon active phase that is coated on $\gamma$-$Al_2O_3$ support, said $\gamma$-$Al_2O_3$ support being a host matrix for said carbon active phase.

"Direct dehydrogenation" refers herein to a chemical reaction that involves the removal of hydrogen from an organic molecule. In direct dehydrogenation, the process is carried out substantially in the absence of oxygen or, in other words, is carried out under oxygen-free conditions.

Dehydrogenation may be applied to any hydrocarbon, and for example to ethylbenzene, ethane, propane, isobutane, normal butane or cyclic alkanes. Dehydrogenation may convert ethylbenzene to styrene, ethane to ethylene, propane to propylene, isobutene to isobutylene, normal butane to n-butene and cyclic alkanes to the corresponding cyclic alkenes. The number of carbons contained in the cyclic alkanes is not limited and thus, virtually all gaseous cyclic alkanes in the range of the reaction temperature described above can be converted into their corresponding alkenes. Preferably, the dehydrogenation reaction of the invention allows converting ethylbenzene (EB) to styrene (ST) according to the following equation:

$$C_6H_5CH_2CH_3 \rightarrow C_6H_5CH=CH_2 + H_2$$

Dehydrogenation reaction of the invention may comprise an initial step consisting in flowing a stream of a feed made at least of one reagent over the catalyst support. During this initial phase the dehydrogenation catalyst may be formed in the reactor and used as such ("*in-situ* method"), or it may be pre-formed ("*ex-situ* method") and then used as dehydrogenation catalyst in the reactor.

In the *"in-situ"* method the carbon layer(s) formed on the $\gamma$-Al$_2$O$_3$ support originates from the decomposition of a gaseous hydrocarbon, especially ethylbenzene, at the surface of the support and directly in the catalyst reactor. The synthesis of the catalyst may be accomplished at a reaction temperature ranging from 400 °C to 750 °C, preferentially from 500 °C to 650 °C, and especially from 550 °C to 600 °C.

In the "*ex-situ*" synthesis a thin layer of carbon species is deposited at the surface of a $\gamma$-Al$_2$O$_3$ support by flowing a stream of a feed made of an hydrocarbon, from $C_1$ to $C_{20}$, preferably from $C_1$ to $C_7$, and especially from $C_2$ to $C_4$, on $\gamma$-Al$_2$O$_3$ at a temperature ranging from 400 °C to 750 °C, preferentially from 550 °C to 700 °C, and especially from 650 °C to 680 °C. The synthesis duration can be performed from 0.5 to 24 h, although the maximum synthesis duration is not limited to 24 h, preferentially from 1 to 6 h, and especially from 2 to 4 h.

Dehydrogenation processes are extensively used in industry and the skilled person will conveniently adapt the reaction parameters to the process of the invention.

The stream of feed containing the hydrocarbon is contacted with the catalyst for an appropriate time and at the most suitable temperature for the dehydrogenation reaction to occur. The stream of feed may contain an inert diluent for the hydrocarbon such as nitrogen or another inert gas. However, steam is not present in the feed stream. Thus, the direct dehydrogenation protocol of the invention is carried out in the absence of steam in the reactant feed. In other words, no steam is injected in the process during the dehydrogenation reaction. The reaction can be carried out for a period exceeding several hundred hours without any evidence of catalyst deactivation. According to the observed results it is expected that the deposited carbon at $\gamma$-Al$_2$O$_3$ surface acts as metal-free dehydrogenation catalyst and no surface fouling with inactive carbon species occurs throughout the process. As mentioned above the carbon deposition onto $\gamma$-Al$_2$O$_3$ surface may also confer to the final composite a higher thermal conductivity than that of pristine $\gamma$-Al$_2$O$_3$. This can significantly reduce the temperature gradient within the catalytic bed throughout the dehydrogenation process, thus allowing the catalyst to maintain the dehydrogenation performance even at high ethylbenzene conversions per pass.

Advantageously, the DDH process of the invention is less performing in the presence of steam (in the reactant feed) compared to the same process realized in the absence of steam. However, it is worthy to note that once steam is removed from the reactant feed the original dehydrogenation performance is rapidly recovered thus confirming the extremely high stability of the catalyst.

Regarding the operative temperature, the reaction may be carried out at a suitable temperature value that may be comprised between 400°C to 700°C, more particularly from 450°C to 600°C, or from 500°C to 600°C. Such a temperature may be reached by heating the reactor and/or the catalyst bed and/or the feed stream to a temperature value sufficient to provide the targeted reaction temperature. The heating is accomplished through a conventional approach known to the skilled person.

The direct dehydrogenation process of the invention may be realized for a period of several months. The direct dehydrogenation may be realized for example for a period of at least 600 hours. Advantageously, the dehydrogenation activity of the catalyst - expressed in terms of hydrocarbon or ethylbenzene conversion as the case may be - is increased (as a function of the test duration) up to reach the steady-state performance in about 20 hours. The dehydrogenation activity, expressed in terms of hydrocarbon or ethylbenzene conversion as the case may be, remains stable for the whole catalytic run thus indicating that deactivation phenomena (i.e. surface fouling with carbonaceous residues or leaching of the active phase) are unlikely to occur. Increasing the reaction temperature leads to an increase of the dehydrogenation activity.

The stream rate may be determined by the person skilled in the art in view of his general knowledge. For example, it may be of about 50 mL/min. However, reactions can also be carried out at higher or lower reactant stream rates.

The direct dehydrogenation reaction may be realized at various hydrocarbon, for example ethylbenzene, concentrations, for example from 2 to 20 vol.%, preferably 3 to 10 vol.%, in the feed stream containing an inert gas as carrier.

One of the advantages of the DDH process of the invention is that it may be realized under reaction conditions close to those used in industrial plants, such as high ethylbenzene concentration, 10 vol.%, or higher, in the reactant feed, and high reaction temperature: for example from 600°C up to 650°C, for a long duration assessment. Unlike previous processes, the process of the invention does not require the addition of steam in the reactant feed.

Another advantage of the DDH process of the invention deals with the extremely high styrene selectivity which is close to 98%. Such styrene selectivity is much higher than that reported for ODH processes as well as for traditional steam-assisted dehydrogenation processes.

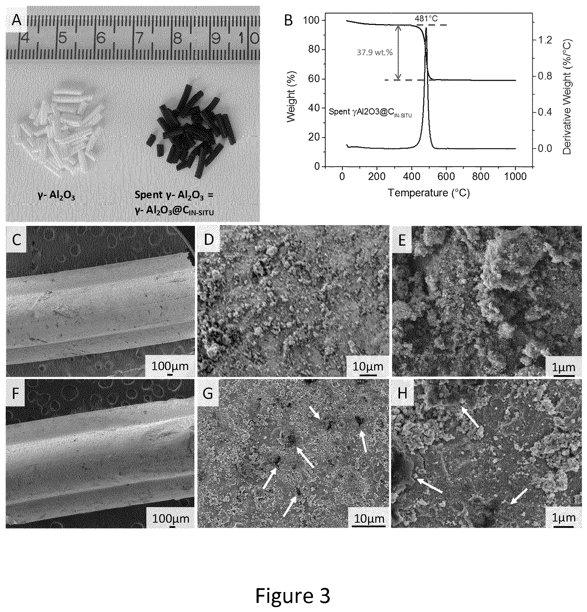

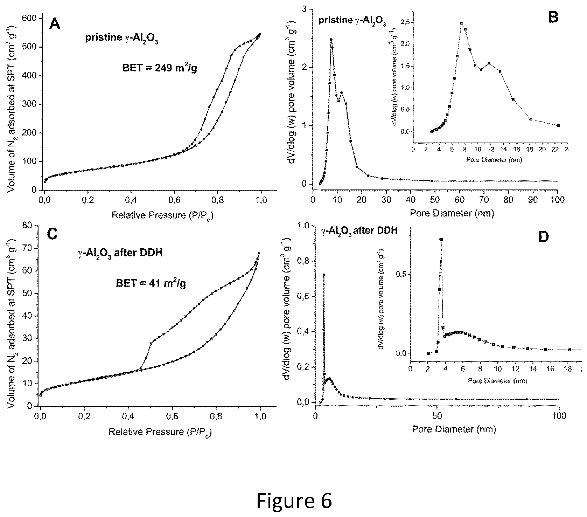

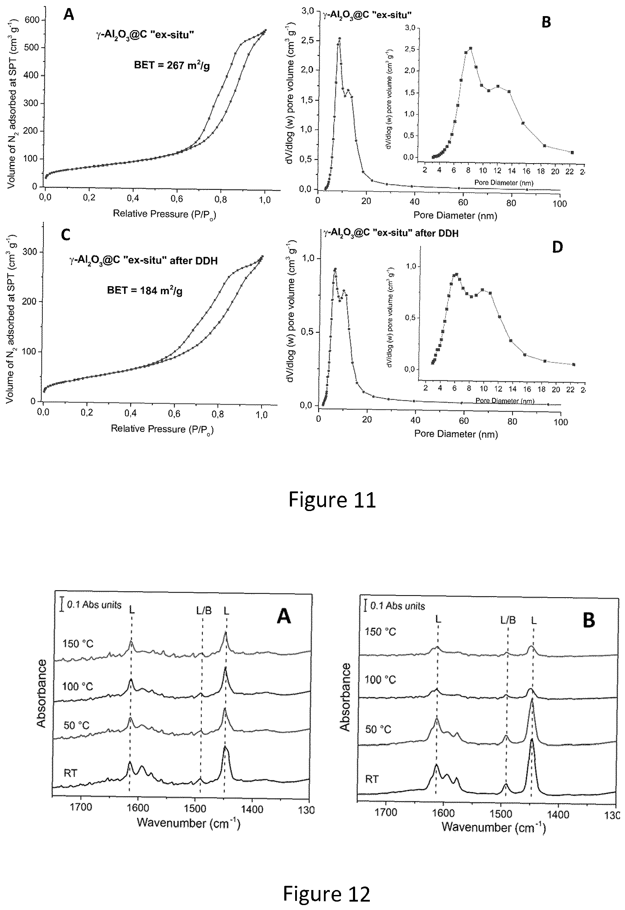

The catalyst of the invention consists of a carbon active phase, which means that it comprises 100% carbon by weight in the active phase. In particular, the catalyst of the invention does not contain metals. Advantageously, the carbon phase is catalytically active for the dehydrogenation reaction. Advantageously, the carbon phase deposited during the *"in-situ"* or *"ex-situ"* process, displays a difference in terms of specific surface area, preferably measured by $N_2$ physiosorption (BET method) at the liquid $N_2$ temperature, and pore size distribution (BJH method). For the « *in-situ* » prepared catalyst ($\gamma$-$Al_2O_3$@$C_{IN-SITU}$), the specific surface area (SSA) may decrease - for example - from 249 $m^2/g$ in the pristine $\gamma$-$Al_2O_3$ to about 41 $m^2/g$ (Figure 6 A *vs.* 6 C) in the C-coated composite (used catalyst) the latter containing about 38 wt.% in carbon (see Figure 3 B). A significant modification of the pore size distribution is observed between pristine $\gamma$-$Al_2O_3$ and its C-coated composite, the latter showing smaller mesopores within a narrower pore-size distribution. (Figure 6 B *vs.* 6 D). On the other hand, once the « *ex-situ* » sample is treated in catalysis ($\gamma$-$Al_2O_3$@$C_{EX-SITU}$ in DDH), BET may vary - for example from 267 $m^2/g$ to 184 $m^2/g$ (Figure 11 A *vs.* 11 C) and pore size distribution is substantially confirmed or only slightly modified (Figure 11 B *vs.* 11D). Accordingly, the nature and microstructure of carbon layers formed during the "*in-situ*" or "*ex-situ*" syntheses are different. In the case of the *"ex-situ"* catalyst the additional C-phase grown during the DDH process with ethylbenzene, generates a surface coating featured by larger mesopores. The different microstructure of the C-phase in the $\gamma$-$Al_2O_3$@$C_{EX-SITU}$ catalyst after DDH is likely related to its higher DDH activity.

A significant difference between the process of the invention and dehydrogenation processes of the state-of-the-art is that no inactive coke deposits are generated throughout the dehydrogenation reaction. Indeed no deactivation of the catalyst takes place in the process of the invention thus avoiding the need of any costly, time- and energy-consuming catalyst reactivation phase (coke removal). Indeed, all carbon deposits formed during the DDH reaction are catalytically active, *i.e.* they contains catalytically active carbon species. Therefore, the dehydrogenation process of the invention operates without requiring any catalyst regeneration step. It is an advantage of the present invention that the process can be operated for more than thousand hours without need of any catalyst regeneration or removal of adventitious carbon deposits. However, in the case of extremely long-term operation procedures, carbon phase deposits can modify the overall catalyst volume thus causing problems of pressure drop in the system. Therefore, long-term used catalysts can be regenerated using mild oxidative treatments under diluted air or steam.

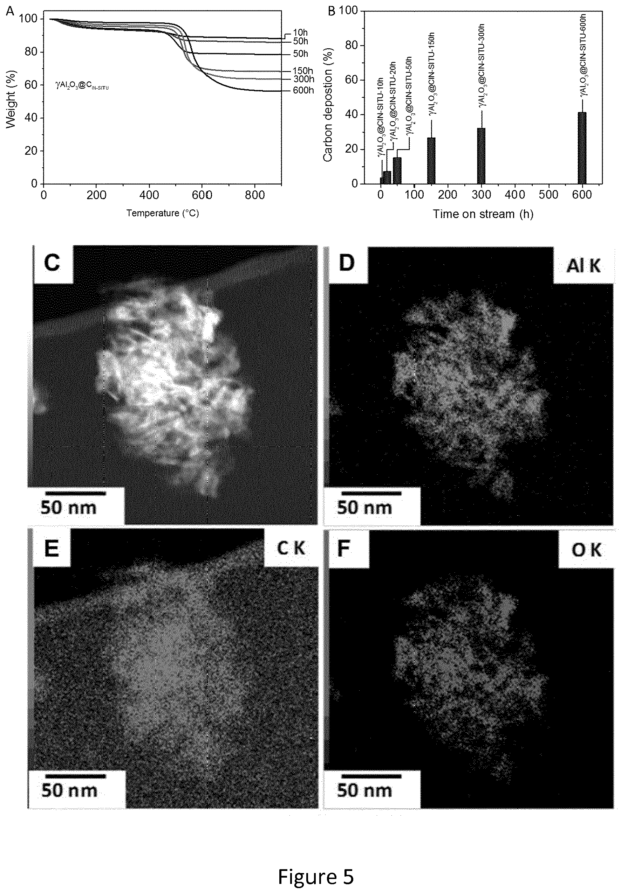

According to the invention, the carbon active phase is coated on a $\gamma$-$Al_2O_3$ support. The $\gamma$-$Al_2O_3$ support may be constituted exclusively of $\gamma$-$Al_2O_3$, which means that the $\gamma$-$Al_2O_3$ support comprises 100 % by weight of $\gamma$-$Al_2O_3$. The $\gamma$-$Al_2O_3$ can also contain some promoters or impurities added throughout its industrial production. The $\gamma$-$Al_2O_3$ support may be a continuous solid phase, which can be produced according to any known industrial synthetic process. The $\gamma$-$Al_2O_3$ support is a host matrix for the carbon active phase. Advantageously, the carbon particles may be embedded or deposited on the $\gamma$-$Al_2O_3$ support. The carbon active phase may coat the surface of the $\gamma$-$Al_2O_3$ support, grown up from the pristine alumina porosity or from the alumina surface as a layer with variable thicknesses. The elemental mapping carried out on the $\gamma$-$Al_2O_3$ based catalyst after 600 h of reaction, confirms the homogeneous coating of the alumina host matrix by layers of carbon (Figures 5C-F). No other elements has been detected in the catalyst active phase.

The support could also be made by different alumina phases, for example a layer of $\gamma$-$Al_2O_3$ coated on $\alpha$-$Al_2O_3$ or other alumina cores or a mixing of different types of alumina structures. The $\gamma$-$Al_2O_3$ support may be a macroscopic support for hosting the carbon active phase or a coating layer on other insulator or thermal conductive supports for hosting the carbon active phase. The thermal conductive support can be for example a coating layer on other organic/inorganic/hybrid supports such as carbon, silicon carbide or others suitable for hosting the carbon active phase deposited afterwards. A colloidal solution (sol) of an alumina precursor may also be coated onto a selected host matrix followed by an appropriate thermal treatment to convert the alumina coating into a $\gamma$-$Al_2O_3$ phase. The latter will be the host matrix of choice to be used for depositing a layer of the active carbon phase for the DDH process. Advantageously, the support for hosting the $\gamma$-$Al_2O_3$ phase can be selected among organic/inorganic/hybrid matrices such silicon carbide, $\alpha$-$Al_2O_3$ or other alumina phases, $TiO_2$, carbon and stainless steel. Advantageously, the use of a second support with higher thermal conductivity, *i.e.* carbon or silicon carbide, may reduce the problem of temperature gradient within the catalyst bed due to the endothermic character of the DDH reaction. The use of a mixture of $\gamma$-$Al_2O_3$ and other alumina structures, such as $\alpha$-$Al_2O_3$, $\theta$-$Al_2O_3$ or other alumina phases, known by the person skilled in the art, can advantageously reduce the overall production cost of the support for the preparation of the targeted catalyst.

The $\gamma$-$Al_2O_3$ can be deposited onto the supports cited above by any other known method, for example through impregnation of a colloidal $\gamma$-$Al_2O_3$ solution (sol) or a suspension of $\gamma$-$Al_2O_3$ powder or through a ball milling process.

The $\gamma$-$Al_2O_3$ support may be in the form of shaped particles. Any suitable shape may be used. For example, it can be used as particles having small dimensions, at least 0.1 mm, preferably of 0.5 mm and especially from 1 to 3 mm. It may be in the form of granules, spheres, cylinders, lobed cylinders, rings, saddles, plates, monolith, foam or any other shape commonly used as catalyst supports for fixed bed applications. The $\gamma$-$Al_2O_3$ can also be associated with other thermal conductive supports such as silicon carbide, carbon or stainless steel, in the form of macroscopically shaped host structures or plates in order to maintain the reaction temperature inside the catalyst bed as much homogeneous as possible. Conductive inert supports could also be physically mixed with the $\gamma$-Al$_2$O$_3$ for regulating the heating phase. The catalyst can also be operated in microreactors where heat and mass transfer phenomena are significantly improved compared to fixed-bed reactor configurations.

The ensemble of carbon active phase and $\gamma$-Al$_2$O$_3$ support are referred hereafter as "composite".

In one embodiment, the catalyst is formed during the direct dehydrogenation reaction by flowing a hydrocarbon gas diluted in an inert gas stream over a $\gamma$-Al$_2$O$_3$ support. In this embodiment, the catalyst is formed "*in-situ*". The reaction temperature may be the same of that used in the dehydrogenation process, *i.e.* from 400°C to 700°C, for a duration that may be comprised between 2 and 30 h, especially between 5 and 20 h.

In another embodiment, the catalyst is formed "*ex-situ*", i.e. before the direct dehydrogenation reaction, by flowing the $\gamma$-Al$_2$O$_3$ support with a stream of a gaseous aliphatic hydrocarbon such as C$_n$H$_{2n+2}$; with "n" typically in the 1-20 range, or aromatic hydrocarbons, diluted in an inert gas, such as helium, nitrogen or argon, at a stream temperature between 500°C and 700°C, for a duration comprised between 2 and 10 h, preferentially between 2 and 6 h and especially between 2 and 4 h. In this embodiment, the catalyst formed *ex-situ* is directly active for the direct dehydrogenation (DDH) of hydrocarbons, for example for the DDH of ethylbenzene (EB).

Another object of the invention deals with a process of preparation of a composite comprising a carbon active phase coating on a $\gamma$-Al$_2$O$_3$ support, said $\gamma$-Al$_2$O$_3$ support being a host matrix for said carbon active phase. This process comprises the step of treating a support consisting of $\gamma$-Al$_2$O$_3$ at a temperature comprised between 550°C and 650°C under an ethane (C$_2$H$_6$) stream for a period of at least 1.5 hours ("*ex-situ* method") or under a stream of ethylbenzene, ethane or directly under ethylbenzene diluted in an inert gas stream ("*in-situ method*").

Regarding the *in-situ* method, all parameters described for the dehydrogenation process of the invention may be applied.

Regarding the *ex-situ* method, the inert gas is selected preferentially among helium, nitrogen and argon. In the "*ex-situ*" catalyst synthesis, the hydrocarbon may be selected among C$_1$ to C$_{20}$, preferentially C$_1$ to C$_7$, and especially C$_2$ to C$_4$.

The stream of feed containing the hydrocarbon is contacted with the catalyst for an appropriate time and at the most suitable temperature for the carbon coating to form at the surface of the support. For example, a sufficient amount of carbon active phase can be formed on the support so that at least 0.5 wt.%, more preferably 3 wt.% of the resulting composite comprises carbon.

The period of contacting the hydrocarbon feed with the support may be at least 1.5 hour, or at least 3 hours, or at least 6 hours, both for the *ex-situ* and *in-situ* method.

For both *in-situ* and *ex-situ* methods, the stream rate may be determined by the person skilled in the art in view of his general knowledge. The reactant(s) flow rate, which contains ethylbenzene (EB) at various concentrations - from 2.8 vol.% up to 10 vol.% - is established in about 90 mL/min per g of catalyst. It is worthy to note that DDH process can also be carried out under different reactant(s) flow rates and EB concentrations.

Another object of the invention deals with a composite comprising a support consisting of $\gamma$-Al$_2$O$_3$ and a layer consisting of a carbon active phase, wherein said $\gamma$-Al$_2$O$_3$ support is a host matrix for said carbon active phase layer, obtainable by an "*ex-situ* method" or during the process of direct dehydrogenation ("*in-situ* method").

Another object of the invention relates to the use of the composite of the invention for the oxygen- and/or steam-free direct dehydrogenation of an hydrocarbon, especially ethylbenzene (EB) to styrene (ST).

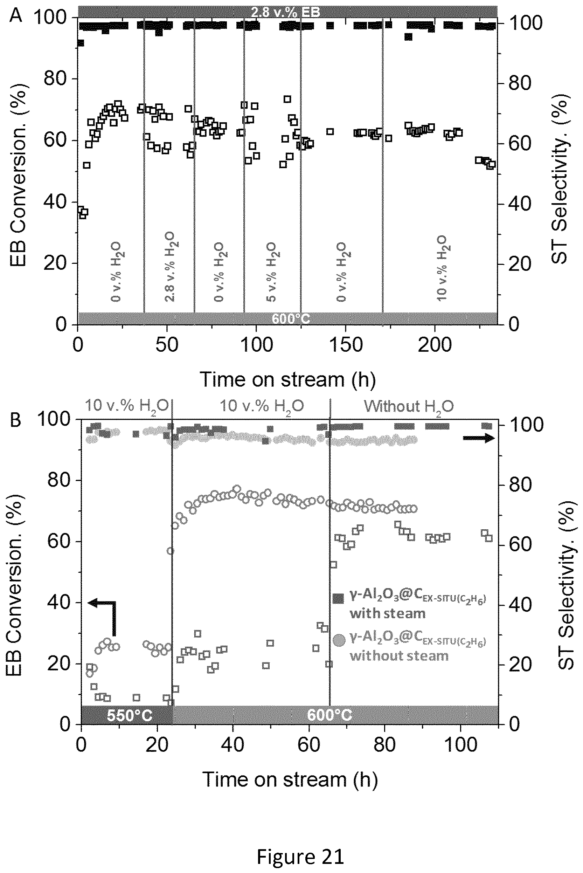

In another variant of the invention the reagent feed can also contain some steam, i.e. 10 vol.%. With the $\gamma$-Al$_2$O$_3$@C$_{EX-SITU}$ catalyst, the presence of steam (as typically occurs in industrial dehydrogenation processes with K-promoted iron oxide catalysts) causes a decrease of the dehydrogenation performance (as illustrated in Figure 21). It can be concluded that steam and ethylbenzene competitively adsorbs at the carbon active phase of the catalyst thus resulting into a reduction of its performance. However, the catalysts remain stable as a function of time-on-stream which indicates that steam did not alter the nature of the catalyst C-active phase. The dehydrogenation performance steadily increases after removal of steam in the reactant feed (Figure 21 B).

This invention is further illustrated by the following examples along with annexed drawings that should not be considered as either unique or limitative for the invention.

## Brief description of the figures

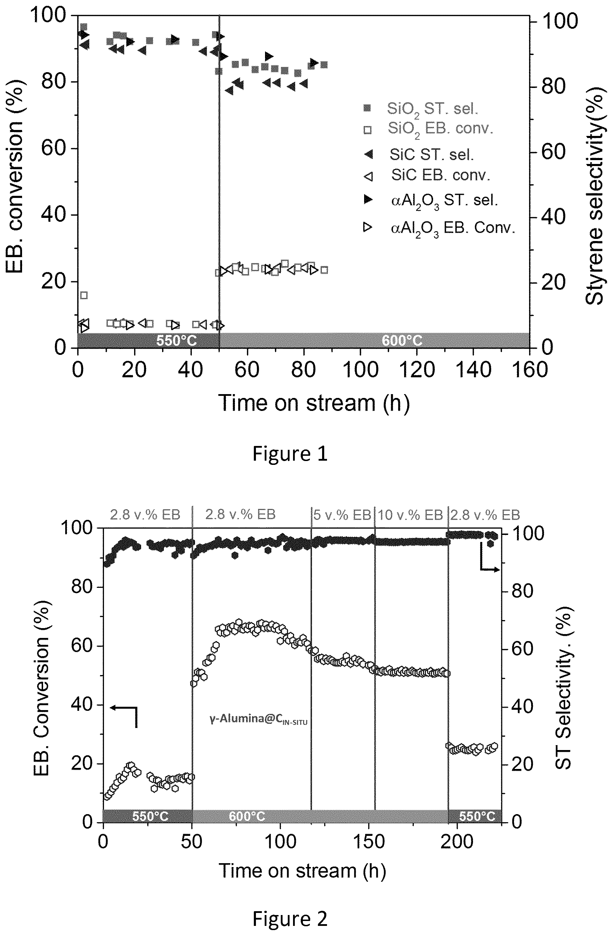

- Figure 1 outlines the catalyst performance in EB dehydrogenation (DDH) to ST by using SiO$_2$, SiC and $\alpha$-Al$_2$O$_3$ as supports for the carbon phase coating. The resulting composites are used as catalysts for the process. EB conversion (C$_{EB}$), and styrene selectivity (S$_{ST}$) are expressed in percentages as a function of the reaction duration (expressed in hours). Empty symbols: EB conversion (square = C$_{EB}$ on SiO$_2$; triangle pointing to left = C$_{EB}$ on SiC; triangle pointing to right = $C_{EB}$ on $\alpha$-$Al_2O_3$). Solid symbols: styrene selectivity (square = $S_{ST}$ on $SiO_2$; triangle pointing to the left = $S_{ST}$ on SiC; triangle pointing to the right = $S_{ST}$ on $\alpha$-$Al_2O_3$). Reaction conditions: mass of catalyst = 0.3 g; reaction temperature ($T_{reaction}$) = 550 and 600 °C, EB concentration [EB] = 2.8v.% in helium, flow rate = 30 mL / min, GHSV (STP) = 3000 h$^{-1}$, atmospheric pressure.

- Figure 2 outlines the performance in EB dehydrogenation (DDH) to ST of a carbon catalyst generated *ex situ* ($\gamma$-$Al_2O_3$@$C_{IN-SITU}$) using $\gamma$-$Al_2O_3$ as support (in the form of extruded trilobes of 1 × 3 mm). EB conversion ($C_{EB}$) and ST selectivity ($S_{ST}$) are expressed in percentage and are reported as a function of the reaction duration (expressed in hours); time on stream of EB is expressed in hours. Empty symbols (circles) = EB conversion ($C_{EB}$); Solid symbols (circles): styrene selectivity ($S_{ST}$). Reaction conditions: mass of catalyst = 0.3 g, reaction temperature ($T_{reaction}$) = 550 and 600 ° C, EB concentration [EB] = 2.8, 5 and 10 vol.% in helium, flow rate = 30 mL / min, GHSV (STP) = 3000 h$^{-1}$, atmospheric pressure.

- Figure 3: **A**: Digital image of $\gamma$-alumina before and after 600 h test. Left side hand image: $\gamma$-$Al_2O_3$; right side hand image: used catalyst $\gamma$-$Al_2O_3$@$C_{IN-SITU}$. **B**: measure of the amount of carbon phase deposited on the $\gamma$-$Al_2O_3$ support by Thermogravimetric Analysis (TGA). The amount of carbon deposit on $\gamma$-$Al_2O_3$ after 200 h of test is in the order of 0.5 g (38% of the initial mass of the catalyst). The weight loss (%) and Differential Thermogravimetric curve (DTG) are reported as a function of the temperature (°C). **C, D, E** and **F, G, H** are SEM micrographs at different magnifications of $\gamma$-$Al_2O_3$ before and after 200 h test, respectively.

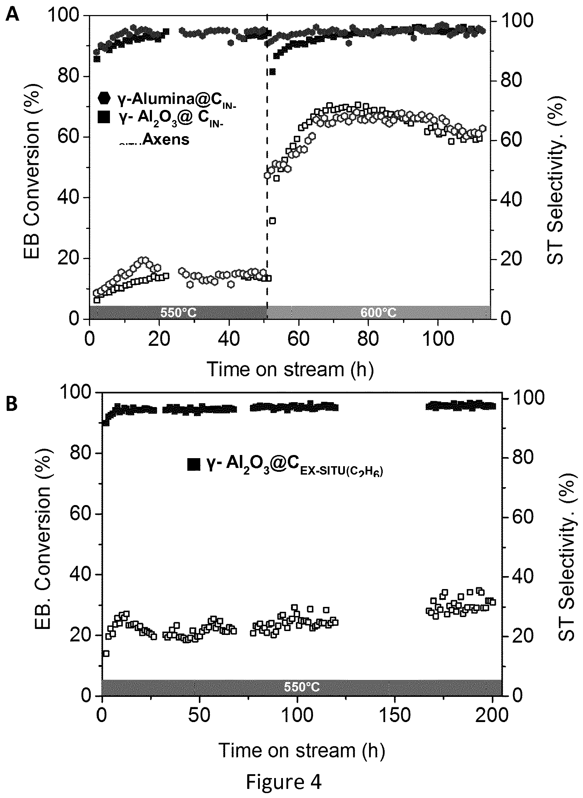

- Figure 4: **A**: Dehydrogenation performance of $\gamma$-$Al_2O_3$@$C_{IN-SITU}$ in terms of ethylbenzene (EB) conversion ($C_{EB}$) and ST selectivity ($S_{ST}$) as obtained using different $\gamma$-$Al_2O_3$ supports (Hexagon: $\gamma$-$Al_2O_3$-Ketjen, Square: $\gamma$-$Al_2O_3$ Axens CR 3S). EB conversions ($C_{EB}$) and ST selectivity ($S_{ST}$) are expressed in percentage; time on stream of EB is expressed in hours. Empty symbols: EB conversion ($C_{EB}$); solid symbols: styrene selectivity ($S_{ST}$). Reaction conditions: mass of catalyst = 0.3 g, reaction temperature ($T_{reaction}$) = 550 and 600 ° C, EB concentration [EB] = 2.8 vol.% in helium, flow rate = 30 mL / min, GHSV (STP) = 3000 h$^{-1}$, atmospheric pressure. **B**: Long-term dehydrogenation performance of $\gamma$-$Al_2O_3$@$C_{EX-SITU}$ (Ketjen) as catalyst. EB conversion ($C_{EB}$) and ST selectivity ($S_{ST}$) are expressed in percentage and are reported as a function of the reaction duration (expressed in hours); time on stream of EB is expressed in hours. Empty symbols (squares): EB conversion ($C_{EB}$); solid symbols (squares): styrene selectivity ($S_{ST}$). Reaction conditions: mass of catalyst = 0.3 g, reaction temperature ($T_{reaction}$) = 550° C, EB concentration [EB] = 2.8 vol.% in helium, flow rate = 30 mL / min, GHSV (STP) = 3000 h$^{-1}$, atmospheric pressure.

- Figure 5: **A, B**: Weight (%) of carbon deposits on $\gamma$-$Al_2O_3$@$C_{IN-SITU}$ composites as a function of the reaction duration, measured by Thermogravimetric Analysis (TGA). Reaction conditions: mass of catalyst = 0.3 g, reaction temperature ($T_{reaction}$) = 600 ° C, EB concentration [EB] = 2.8 vol.% in helium, Flow rate = 30 mL / min, GHSV (STP) = 3000 h$^{-1}$, atmospheric pressure. **C-F.** Energy-Electron-Loss Spectroscopy coupled with scanning Transmission Electron Microscopy (EELS-STEM) analysis for the elemental mapping of the $\gamma$-$Al_2O_3$@$C_{IN-SITU}$ catalyst after DDH reaction at 600°C. Pictures show a homogeneous C-coating of the alumina surface.

- Figure 6: **A-D**: Specific surface area (BET method) and pore-size distribution (BJH method) measurements on a $\gamma$-$Al_2O_3$ sample before and after DDH catalysis. Measurements are accomplished using $N_2$ as adsorbent at the liquid $N_2$ temperature. **A** and **C**: $N_2$ Adsorption-desorption isotherms and specific surface area (SSA, expressed in m$^2$/g) as measured before and after DDH, respectively: **B** and **D**: pore size distribution (nm) as measured before and after DDH, respectively.

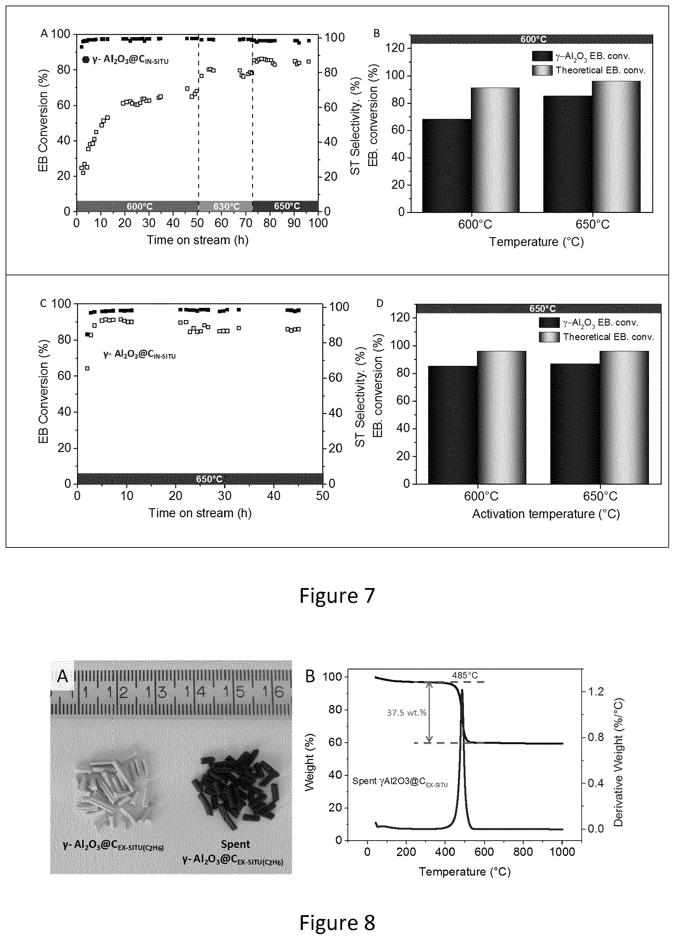

- Figure 7: Dehydrogenation performance of $\gamma$-$Al_2O_3$@$C_{IN-SITU}$ as DDH catalyst expressed as ethylbenzene (EB) conversion ($C_{EB}$) and styrene (ST) selectivity ($S_{ST}$) and outlined as function of the reaction temperature. EB conversion ($C_{EB}$) and ST selectivity ($S_{ST}$) are expressed in percentage and are reported as a function of the reaction duration (expressed in hours); time on stream of EB is expressed in hours. Empty symbols: EB conversion ($C_{EB}$), solid symbols: styrene selectivity ($S_{ST}$). A: $\gamma$-$Al_2O_3$@$C_{IN-SITU}$ activated at 600°C and further tested at different reaction temperatures, i.e. 630 and 650°C, and **B**: DDH performance of $\gamma$-$Al_2O_3$@$C_{IN-SITU}$ activated at 600°C (black headline) expressed in terms of EB conversion ($C_{EB}$) and benchmarked with the theoretical EB conversion (%). Reaction conditions: mass of catalyst = 0.3 g, reaction temperature ($T_{reaction}$) = 600 and 650 °C, activation temperature 600°C, Ethylbenzene concentration [EB] = 2.8 vol.% in helium, flow rate = 30 mL / min, GHSV (STP) = 3000 h$^{-1}$, atmospheric pressure. **C**: $\gamma$-$Al_2O_3$@$C_{IN-SITU}$ activated at 650°C. Reaction conditions: mass of catalyst = 0.3 g, reaction temperature ($T_{reaction}$) = 650 ° C, Activation temperature ($T_{activation}$) = 650°C, Ethylbenzene concentration [EB] = 2.8 vol.% in helium, flow rate = 30 mL / min, GHSV (STP) = 3000 h$^{-1}$, atmospheric pressure. **D**: DDH performance of $\gamma$-$Al_2O_3$@$C_{EX-SITU}$ activated at 650°C (black headline) expressed in terms of EB conversion ($C_{EB}$) and benchmarked with the theoretical EB conversion (%). Reaction conditions: mass of catalyst = 0.3 g, reaction temperature ($T_{reaction}$) = 600 and 650 °C, activation temperature 650°C, ethylbenzene concentration [EB] = 2.8 vol.% in helium, flow rate = 30 mL / min, GHSV (STP) = 3000 h$^{-1}$, atmospheric pressure.

- Figure 8: **A**: digital images of $\gamma$-$Al_2O_3$@$C_{EX-SITU}$ before and after 600 h of testing. Left side hand image: $\gamma$-$Al_2O_3$@$C_{EX-SITU}$; right side hand image: used $\gamma$-$Al_2O_3$@$C_{EX-SITU}$. **B**: measure of the amount of carbon phase deposited on the $\gamma$-$Al_2O_3$ support as estimated via Thermogravimetric Analysis (TGA). The amount of carbon deposited on the catalyst after 600 hours of test is 0.5 g (37.0% of the initial mass of the catalyst). The weight loss (%) and Differential Thermogravimetric curve (DTG) are reported as a function of the temperature (°C).

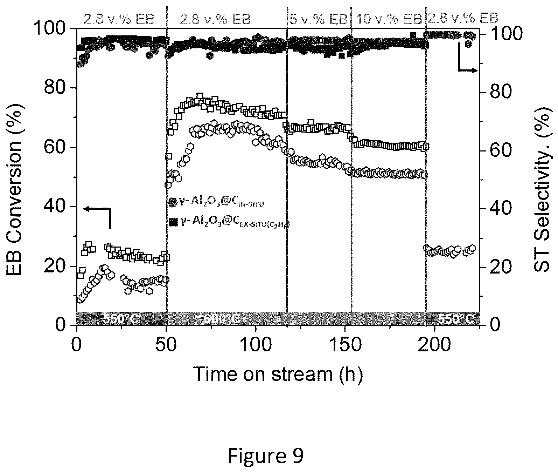

- Figure 9: DDH performance for the EB conversion into ST using an *ex-situ* generated catalyst ($\gamma$-Al$_2$O$_3$@C$_{EX-SITU}$ with $\gamma$-Al$_2$O$_3$ in the form of extruded trilobes of 1 × 3 mm) in comparison with the *in-situ* generated counterpart ($\gamma$-Al$_2$O$_3$@C$_{IN-SITU}$). EB conversion (C$_{EB}$) and ST selectivity (S$_{ST}$) are expressed in percentage and are reported as a function of the reaction duration (expressed in hours); time on stream of EB is expressed in hours. Empty symbols: EB conversion (C$_{EB}$); Solid symbols: styrene selectivity (S$_{ST}$). Reaction conditions: mass of catalyst = 0.3 g, reaction temperature (T$_{reaction}$) = 550 and 600 ° C, Ethylbenzene concentrations [EB] = 2.8, 5 and 10 vol.% in helium, flow rate = 30 mL / min, GHSV (STP) = 3000 h$^{-1}$, atmospheric pressure.

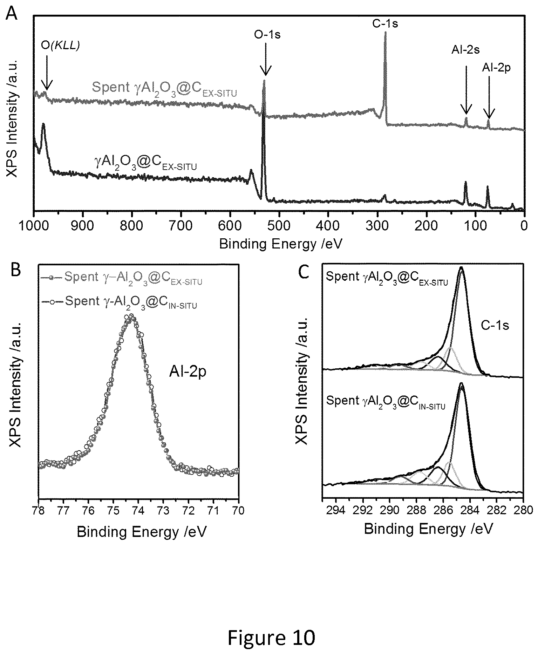

- Figure 10: X-ray photoelectron spectroscopy (XPS) measurements carried out in ultrahigh vacuum (UHV) using a spectrometer equipped with a VSW Class WA hemispherical electron analyzer. **A**: survey spectra showing a general view of different elements detected in the samples: the spectra refer to the $\gamma$-Al$_2$O$_3$@C$_{EX-SITU}$ composite before and after DDH and show an increase of the carbon deposit on the alumina surface for the used catalyst, while peaks of Al and O invariably decrease due to the coverage by the carbon layers. **B** and **C**: High-resolution Al-2p and C-1s XPS spectra of used $\gamma$-Al$_2$O$_3$@C$_{IN-SITU}$ and $\gamma$-Al$_2$O$_3$@C$_{EX-SITU}$ samples put at comparison.

- Figure 11: **A-D**: Specific surface areas (BET method) and pore-size distribution (BJH method) measurements of $\gamma$-Al$_2$O$_3$@C$_{EX-SITU}$ sample before and after DDH catalysis. Measurements are accomplished using N$_2$ as adsorbent at the liquid N$_2$ temperature. **A** and **C**: N$_2$ adsorption-desorption isotherm and specific surface area (SSA, expressed in m$^2$/g) as measured before and after DDH, respectively: **B** and **D**: pore size distribution (nm) as measured before and after DDH, respectively.

- Figure 12: Titration of the Lewis acid groups and Bronsted acid functionalities for a $\gamma$-Al$_2$O$_3$ sample pre-heated at 150 °C (**A**) and 550 °C (**B**), using variable temperature Fourier-transform infrared spectroscopy measurements.

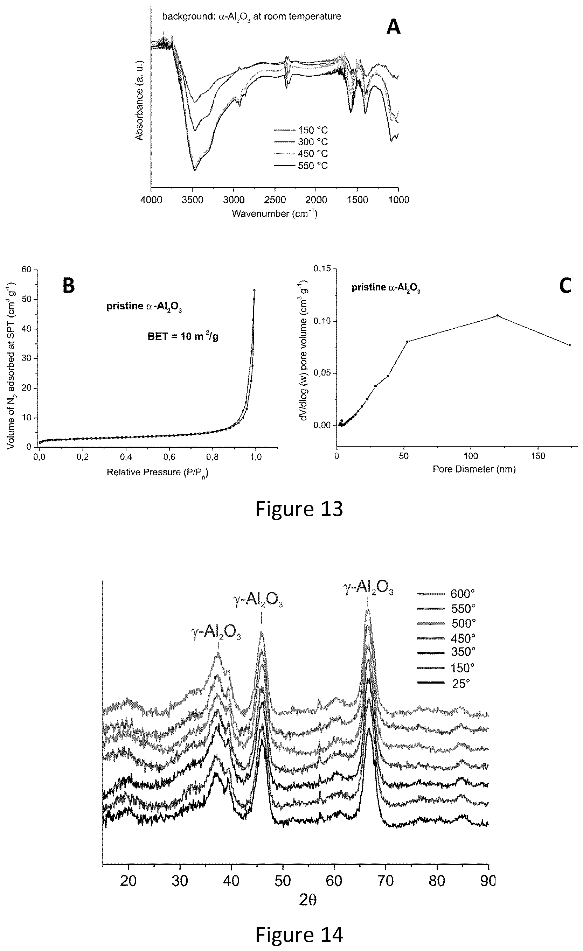

- Figure 13: **A** : Titration of the Lewis acid groups and Bronsted acid functionalities of a $\alpha$-Al$_2$O$_3$ sample after thermal pre-treatment at 150 °C, 300, 450 and 550 °C, respectively. Measurements have been accomplished using variable temperature Fourier-transform infrared spectroscopy. **B** and **C** refer to the specific surface area (BET method) and the pore size distribution (BJH method) on a $\alpha$-Al$_2$O$_3$ sample. Measurements are carried out using N$_2$ as adsorbent at the liquid N$_2$ temperature.

- Figure 14: Investigation of the crystallinity of the $\gamma$-Al$_2$O$_3$ sample as a function of the heating temperature, using an *in-situ* variable temperature powder X-ray diffraction method.

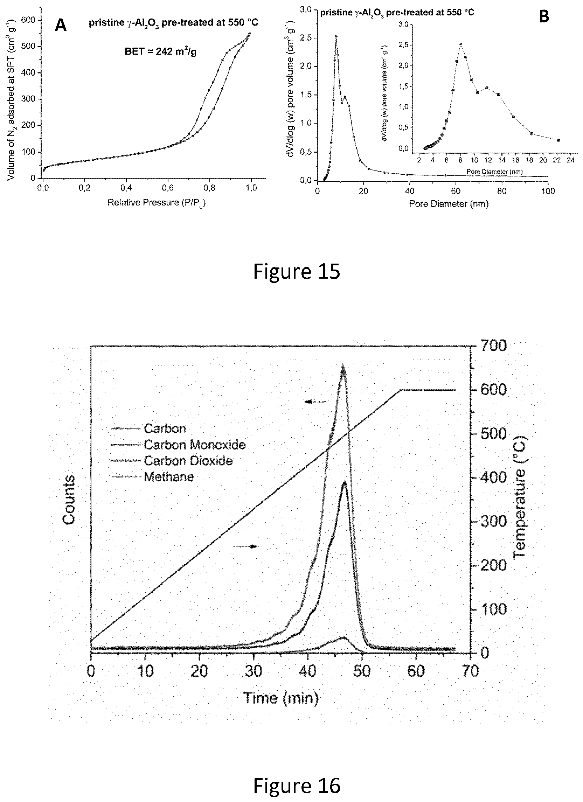

- Figure 15: **A**, **B**: Specific surface areas (BET method) and pore-size distribution (BJH method) of a $\gamma$-Al$_2$O$_3$ sample pre-treated at 550 °C. Measurements are accomplished using N$_2$ as adsorbent at the liquid N$_2$ temperature. **A**: N$_2$ adsorption-desorption isotherm and specific surface area (SSA, expressed in m$^2$/g). **B**: its pore size distribution (nm).

- Figure 16: decomposition products recorded at the outlet of a temperature programmed oxidation (TPO) analyzer, resulting from the thermal decomposition of the carbon deposit at the $\gamma$-Al$_2$O$_3$ support. TPO analysis is conducted in the r.t. - 1100 °C temperature range, under air atmosphere.

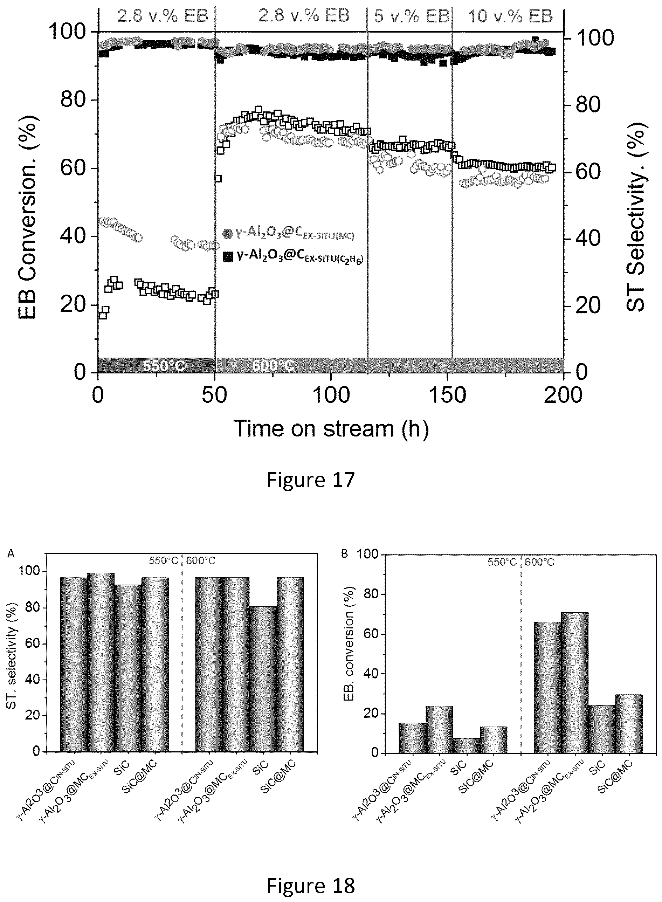

- Figure 17: DDH performance for the EB conversion into ST using an *ex-situ* generated catalyst ($\gamma$-Al$_2$O$_3$@MC$_{EX-SITU}$ with $\gamma$-Al$_2$O$_3$ in the form of extruded trilobes of 1 × 3 mm) where the MC$_{EX-SITU}$ coating is a mesoporous C-layer obtained from impregnation of $\gamma$-Al$_2$O$_3$ in an aqueous mixture of dextrose and citric acid, followed by a thermal treatment under nitrogen atmosphere at 600°C ($\gamma$-Al$_2$O$_3$@MC$_{EX-SITU}$) and compared with another *ex-situ* sample prepared with ethane as stream reagent ($\gamma$-Al$_2$O$_3$@C$_{EX-SITU(C2H6)}$). EB conversion (C$_{EB}$) and ST selectivity (S$_{ST}$) are expressed in percentage and are reported as a function of the reaction duration (expressed in hours); time on stream of EB is expressed in hours. Empty symbols: EB conversion (C$_{EB}$), solid symbols: styrene selectivity (S$_{ST}$). Reaction conditions: mass of catalyst = 0.3 g, reaction temperature (T$_{reaction}$) = 550 and 600 ° C, Ethylbenzene concentrations [EB] = 2.8, 5 and 10 vol.% in helium, flow rate = 30 mL / min, GHSV (STP) = 3000 h$^{-1}$, atmospheric pressure.

- Figure 18: **A, B**: Styrene selectivity (S$_{ST}$) and EB conversion (C$_{EB}$) as obtained on various catalysts at 550 and 600°C with an EB concentration [EB] of 2.8 vol. %. The various catalysts are, from left to right: $\gamma$-Al$_2$O$_3$@C$_{IN-SITU}$, $\gamma$-Al$_2$O$_3$@MC$_{EX-SITU}$, SiC, SiC@MC$_{EX-SITU}$. EB conversion (C$_{EB}$) and styrene selectivity (S$_{ST}$) are expressed in percentage; time on stream of EB is expressed in hours. Reaction conditions: mass of catalyst = 0.3 g, reaction temperature (T$_{reaction}$) = 550 and 600 °C; ethylbenzene concentration [EB] = 2.8 vol.% in helium, flow rate = 30 mL / min, GHSV (STP) = 3000 h$^{-1}$, atmospheric pressure.

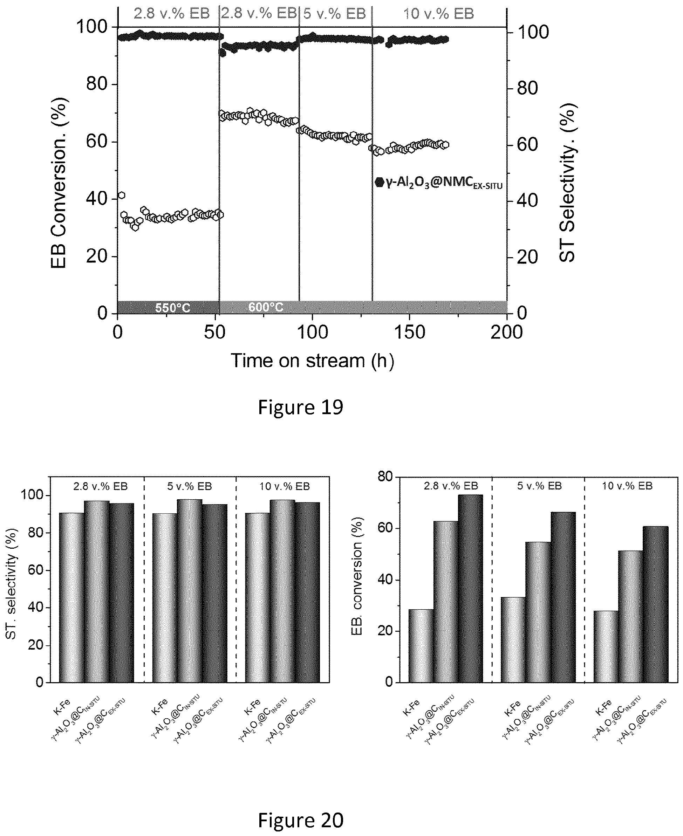

- Figure 19: DDH performance for the EB conversion into ST using an *ex-situ* generated catalyst composed of a nitrogen-doped carbon phase (coating) on $\gamma$-Al$_2$O$_3$ ($\gamma$-Al$_2$O$_3$@NMC$_{EX-SITU}$ with $\gamma$-Al$_2$O$_3$ in the form of extruded trilobes of 1 × 3 mm). A similar procedure to that describe in Figure 17 has been used to generate the NMC coating at the surface of $\gamma$-Al$_2$O$_3$) except for the addition of ammonium carbonate in the dextrose/citric acid aqueous mixture. EB conversion (C$_{EB}$) and styrene selectivity (S$_{ST}$) are expressed in percentage; time on stream of EB is expressed in hours. Empty symbols: EB conversion (C$_{EB}$), solid symbols: styrene selectivity (S$_{ST}$). Reaction conditions: mass of catalyst = 0.3 g, reaction temperature (T$_{reaction}$) = 550-600 ° C, Ethylbenzene concentrations [EB] = 2.8, 5 and 10 vol.% in helium, flow rate = 30 mL / min, GHSV (STP) = 3000 h$^{-1}$, atmospheric pressure.

- Figure 20: **A, B**: Styrene selectivity (S$_{ST}$) and EB conversion (C$_{EB}$) as obtained on various catalysts at 600°C and at various EB concentrations [EB] of 2.8, 5 and 10 vol.%. The catalysts are, from left to right: commercial K-Fe, $\gamma$-Al$_2$O$_3$@C$_{IN\text{-}SITU}$ and $\gamma$-Al$_2$O$_3$@C$_{EX\text{-}SITU}$. Reaction conditions: mass of catalyst = 0.3 g, reaction temperature (T$_{reaction}$) = 600 ° C, Ethylbenzene concentrations [EB] = 2.8, 5 and 10 vol. % in helium, flow rate = 30 mL / min, GHSV (STP) = 3000 h$^{-1}$, atmospheric pressure.

- Figure 21: DDH performance for the EB conversion into ST with and without steam on an *ex-situ* generated catalyst using $\gamma$-Al$_2$O$_3$ as support ($\gamma$-Al$_2$O$_3$@C$_{EX\text{-}SITU}$ with $\gamma$-Al$_2$O$_3$ in the form of extruded trilobes of 1 $\times$ 3 mm). EB conversion (C$_{EB}$) and styrene selectivity (S$_{ST}$) are expressed in percentage; time on stream of EB is expressed in hours. **A**: Empty symbols: EB conversion (C$_{EB}$); Solid symbols: styrene selectivity (S$_{ST}$). The catalyst was activated under steam-free conditions followed by a test in the presence of steam followed by a control test carried under steam-free conditions and so on. According to the results the catalyst, after an activation under steam-free conditions, remains relatively stable as increasing gradually the steam feed in the reactant mixture (from left to right: 0 vol.% H$_2$O, 2.8 vol.% H$_2$O, 0 vol. % H$_2$O, 5 vol.% H$_2$O, 0 vol.% H$_2$O, 10 vol.% H$_2$O). Reaction conditions: mass of catalyst = 0.3 g, reaction temperature (T$_{reaction}$) = 600 °C, Ethylbenzene concentration [EB] = 2.8 vol.% in helium, flow rate = 30 mL·min$^{-1}$, atmospheric pressure. **B**: Empty symbols: EB conversion (C$_{EB}$), solid symbols: styrene selectivity (S$_{ST}$). (Square symbols, $\gamma$-Al$_2$O$_3$@C$_{EX\text{-}SITU(C2H6)}$ with steam): The catalyst was directly activated with 10 vol.% of steam at 550°C and then the reaction temperature was increased to 600°C under steam. According to the observed results the direct activation of the catalyst under steam leads to a significant loss of the DH performance at 550°C and 600°C. The DH performance steadily increases as steam was removed from the reactant feed and the EB conversion increase from 30% (at 600°C and under 10% of H2O) to 60% and remains stable. Circles: $\gamma$-Al$_2$O$_3$@C$_{EX\text{-}SITU(C2H6)}$ without steam. The DH performance of the same catalyst activated and tested under steam-free conditions is reported in Figure 21B for comparison. According to the results the same catalyst activated and tested under steam-free conditions displays a significantly higher DH performance compared to the same activated under steam. Reaction conditions: 550 and 600 °C, 2.8% EB and 10% H$_2$O in helium, flow rate = 30 mL·min-1, atmospheric pressure.

**Examples**

**Example 1: Catalytic dehydrogenation of EB to ST**

The catalytic dehydrogenation of EB to ST is carried out in a fixed-bed continuous flow reactor under atmospheric pressure. The catalyst (300 mg) is loaded onto a quartz fritted disk located inside a tubular quartz reactor (id x l: 8 mm x 800 mm). Helium gas was fed into the reactor at a flow rate of 30 mL min$^{-1}$ by means of a mass flow controller (BROOKS MFC) and passed through a glass evaporator filled with liquid EB maintained at 40°C (EB partial pressure of 2922 Pa) using a thermal regulated bath. The helium flow containing EB was passed downward through the catalyst bed. The concentration of EB in the feed can be modified by changing the temperature of the glass evaporator.

The reaction system was heated to 550-650°C and kept for 1h under He. The reagent flow (2.8 vol. % of ethylbenzene (EB) diluted in helium, total flow rate of 30 mL·min$^{-1}$) was then fed into the reactor. The same procedure is used regardless the catalyst preparation method, i.e. *in-situ* or *ex-situ.* The reagent and the products (styrene (ST), benzene (BZ) and toluene (TOL)) are collected at the reactor outlet and analyzed on-line with a PERICHROM (PR 2100) gas chromatography equipped with a flame detector (FID) and CP WAX S2CB column which was previously calibrated. In order to avoid any possible condensation of the reagents and products all the tube lines were wrapped with a heating wire maintained at 110°C.

The ethylbenzene conversion (X$_{EB}$), styrene selectivity (S$_{ST}$) and reaction yields (Y$_{ST}$) were evaluated using the following equations:

$$X_{EB} = \frac{F_0 C_{EB,inlet} - F C_{EB,outlet}}{F_0 C_{EB,inlet}} \times 100\% \qquad (1)$$

$$S_{ST} = \frac{C_{ST,outlet}}{C_{ST,outlet} + C_{TOL,outlet} + C_{BZ,outlet}} \times 100\% \qquad (2)$$

$$Y_{ST} = X_{EB} \times S_{ST} \qquad\qquad (3)$$

Where F and $F_0$ are the flow rates at the outlet and inlet, respectively, and $C_{EB}$, $C_{ST}$, $C_{TOL}$ and $C_{BZ}$ represent the concentration of ethylbenzene, styrene, toluene and benzene, respectively.

**Example 2: $\gamma$-Al$_2$O$_3$ activation for the preparation of a $\gamma$-Al$_2$O$_3$@C$_{INSITU}$ catalyst**

In this process a known amount of $\gamma$-Al$_2$O$_3$ (300 mg) is loaded onto a quartz reactor (id x l: 8 mm x 800 mm). The reactor was heated to 550-650 °C depending to the targeted activation temperature and then the reagent flow (2.8 vol. % of ethylbenzene (EB) diluted in helium at a total flow rate of 30 mL·min$^{-1}$) was introduced inside the reactor to start the process. The carbon active phase is progressively formed during this treatment and the process is named *in-situ* activation.

**Example 3: $\gamma$-Al$_2$O$_3$ activation for the preparation of a $\gamma$-Al$_2$O$_3$@C$_{EXSITU}$ catalyst**

In this process a known amount of $\gamma$-Al$_2$O$_3$ (1 g) was loaded inside a quartz reactor and flushed with an inert gas (Ar) at room temperature for 30 minutes. Afterwards, the sample was heated under an argon flow from room temperature to 600 °C at a heating rate of 10 °C/min. At 600 °C the argon flow was replaced by a mixture containing $C_2H_6$ and argon ($C_2H_6$ concentration of 10 vol.%) and the treatment was maintained for 2 h.

In another route, the *ex-situ* coated carbon catalyst was straightforwardly prepared by impregnation of $\gamma$-Al$_2$O$_3$ support (5g) with a homogeneous aqueous solution made of dextrose (2g) and citric acid (2g), followed by two successive thermal treatments in air (130 °C) and argon (600 °C); the first treatment is used to dry the support thus forming a tiny pre-catalytic layer; the second thermal heating (heating rate of 10 °C/min) allows to generate the catalytically active C-phase as a coating of the $\gamma$-Al$_2$O$_3$ matrix.

**Example 4 : Study of the direct dehydrogenation (DDH) activity of carbonaceous structures deposited on different supports: silica, silicon carbide or alumina ($\alpha$-Al$_2$O$_3$)**

The supports used in these tests are of three types: silica (SiO$_2$), silicon carbide ($\beta$-silicon carbide) and alpha-alumina ($\alpha$-Al$_2$O$_3$).

The DDH tests are carried out as a function of reaction time using an EB concentration [EB] of 2.8 vol.% in helium and in the absence of steam. Catalytic texts have shown that very moderate carbon phases are formed during the DDH reaction on the studied supports (SiO$_2$, SiC or $\alpha$-Al$_2$O$_3$), thus resulting in relatively low EB conversions ($C_{EB}$) and styrene selectivity ($S_{ST}$), especially at reaction temperatures approaching 600 °C (Figure 1).

**Example 5: DDH on *in situ* formed C-phases using $\gamma$-Al$_2$O$_3$ as support**

Results obtained in the case of $\gamma$-Al$_2$O$_3$ as support for the preparation of $\gamma$-Al$_2$O$_3$@C$_{IN-SITU}$ catalyst have shown that DDH activity increases with time under the reactant flow at 550°C, up to reach a plateau with an EB conversion ($C_{EB}$) of about 15% and a styrene selectivity ($S_{ST}$) in the order of 98%. The DDH activity obtained on the catalyst $\gamma$-Al$_2$O$_3$@C$_{IN-SITU}$ increases significantly when the reaction temperature increases from 550 to 600 °C while keeping all other reaction conditions similar (Figure 2). It should be noticed that the reaction conditions, T = 600 °C and EB concentration ([EB] = 10 vol.%), are relatively close to those commonly used in industrial plants for this process but without the presence of any water vapor (steam). Styrene production remains relatively high, around 52%, with a styrene selectivity ($S_{ST}$) in the order of 98%, whatever the reaction conditions used. It is also worthy to note that the catalyst reaches a steady-state after the activation step and no deactivation takes place. This result highlights the robustness of the catalytic materials and their high resistance towards deactivation by coke fouling or active phase degradation.

The deposition of native carbon during the DDH reaction was observed due to the significant change in the color of the catalyst support, before and after testing (Figure 3A). According to SEM results we can observe that the surface of $\gamma$-Al$_2$O$_3$@C$_{IN-SITU}$ (Figure 3F, G and H) catalyst is less rough compare to that of the pristine support ($\gamma$-Al$_2$O$_3$) (Figure 3C, D and E), and *in-situ* formed dark deposits of carbon are present. The amount of carbon phase deposited on $\gamma$-Al$_2$O$_3$ support was analyzed by Thermogravimetric Analysis (TGA) and the results are presented in Figure 3B.

It should be also noticed how the activity of the catalyst is significantly modified after increasing the initial reaction temperature from 550 to 600°C and under different EB concentrations. Indeed, a "return test" at 550 °C shows that the conversion of the EB ($C_{EB}$) is passed from 15% to about 26% on the same catalyst as a consequence of the previous temperature increase at 600 °C. Such a results can be explained by the fact that the carbon species formed at 600 °C is much "active" for the EB DDH process than that generated *in-situ* at 550 °C.

The same tests were carried out on different $\gamma$-Al$_2$O$_3$ supports featuring more opened porous structures. The results obtained confirm the formation of a native carbon layer active for the DDH reaction of EB to ST. From the results obtained, it seems to be confirmed that only the $\gamma$-Al$_2$O$_3$ support is active to form the carbon layer active (as metal-free system) in the steam-free DDH reaction of EB to ST. Indeed, the results presented above (see example 4 and Figure 1) on $\alpha$-Al$_2$O$_3$ as support, confirm that the active carbon layer was not (or only slightly) formed on the latter. It can be inferred that the different behavior of the two alumina supports is likely due to their different specific surface area and porosity as well as their different surface concentration of acid groups, and more specifically Lewis acid groups. Lewis acid groups available on $\gamma$-Al$_2$O$_3$ are much more numerous than those available on $\alpha$-Al$_2$O$_3$. Accordingly, the "active carbon layer" is not formed either at the surface of silica (SiO$_2$) or SiC (the surface of the latter is coated by a thin layer of silica) which contains less Lewis acid groups.

Thus, the inventors have shown that the native carbon generated *in-situ* at the surface of $\gamma$-Al$_2$O$_3$ is active and relatively stable for the steam-free direct dehydrogenation (DDH) of EB into ST. The obtained ST yields are extremely promising for further industrial exploitation of the DDH process without the use of water vapors (steam). The absence of steam is particularly relevant from an industrial viewpoint and it represents an important incentive to the process industrialization. Indeed, extra energy input and process costs for generating steam are no longer required and large amounts of wastes are additionally avoided (water exit from the reactor with trace amounts of hydrocarbons).

Two different $\gamma$-Al$_2$O$_3$ samples were tested under identical conditions without showing appreciable differences. The two-tested catalysts showed almost the same catalytic performance at reaction temperatures of 550-600°C (Figure 4A). They have shown high stability (over more than 200 hours under catalysis) with no deactivation. On the contrary, catalytic performance were found to increase (slightly) as a function of time-on-stream. A test carried out on $\gamma$-Al$_2$O$_3$@C$_{EXSITU}$ at 550°C for prolonged reaction times, indicates that DDH activity slightly increases during the whole test which could be attributed to the formation of a carbon active phase with higher active sites density (Figure 4B).

The porosity of the $\gamma$-Al$_2$O$_{3@}$C$_{IN\text{-}SITU}$ before and after DDH test is shown in Figure 6. The sample obtained after DDH (Figure 6C and D) presents a significant reduction of SSA (from 249 m$^2$/g to 41 m$^2$/g). This can be reasonably expected as a consequence of the carbon deposit and pores clogging. It is evident that after DDH a prevalent and narrow distribution of pores is available in the 3-4 nm range (Figure 6D).

**Example 6: Direct dehydrogenation on *in-situ* carbon catalyst supported on $\gamma$-Al$_2$O$_3$ as a function of the activation temperature**

The results reported in this example describe the variation of DDH performance as a function of the activation temperature. In this example $\gamma$-Al$_2$O$_3$ was loaded inside the reactor and activated under a flow of EB in helium (EB concentration of 2.8 vol.%) at different temperatures, i.e. 600°C and 650°C. The DDH results are presented in Figure 7 as a function of time-on-stream. The catalytic outcomes recorded with the $\gamma$-Al$_2$O$_{3@}$C$_{IN\text{-}SITU}$ catalyst confirms again the very high DDH performance and stability under different activation temperature, i.e. 600 and 650 (Figure 7A and C). The activation of the catalyst directly at 650°C significantly reduces the time to reach the steady-state and the DH performance obtained at the same temperature, i.e. 650°C, is similar. The obtained activities are close to the predicted theoretical values (Figure 7B and D). According to Figure 7B and D, the activation temperature of 600°C or 650°C does not show any significant influence on the EB conversion at high temperature, i.e. 650°C (right part of Figure 7B and D), while the high activation temperature, i.e. 650°C, slightly improve the EB conversion when the catalyst was operated at lower reaction temperature, i.e. 600°C (left part of Figure 7B and D).

**Example 7: Direct dehydrogenation (DDH) on *ex-situ* carbon catalyst supported on $\gamma$-Al$_2$O$_3$**

The previous examples have shown that an active *in-situ* carbon phase for the DDH reaction of EB into ST was produced only at the surface of $\gamma$-Al$_2$O$_3$ as support.

In this example we have investigated the activity of a layer of carbon deposited by an *ex-situ* method at the surface of $\gamma$-Al$_2$O$_3$ for the DDH reaction of EB into ST. The *ex-situ* carbon layer is generated by treating the $\gamma$-Al$_2$O$_3$ support at 600°C for 2 h with an ethane stream (50mL/min) diluted with 50 mL/min of argon. The catalyst thus formed is conveniently handled on air and then tested for the DDH of EB into ST.

The purpose of this test is to better control the formation of the active carbon phase directly on the support as well as to better control the whole synthetic process for further developments. The synthesized catalyst is denoted $\gamma$-Al$_2$O$_3$@C$_{EX\text{-}SITU}$.

The catalyst denoted $\gamma$-Al$_2$O$_3$@C$_{EX\text{-}SITU}$ presents a light grey color (Figure 8 A, left) compared to the white color of pristine $\gamma$-Al$_2$O$_3$ in Figure 3A.

Unlike the *in-situ* prepared $\gamma$-Al$_2$O$_3$ catalyst ($\gamma$-Al$_2$O$_3$@C$_{IN\text{-}SITU}$), the $\gamma$-Al$_2$O$_3$@C$_{EX\text{-}SITU}$ exhibits a relatively high DDH activity already in the first minutes of reaction (Figure 9). The DDH activity of $\gamma$-Al$_2$O$_3$@C$_{EX\text{-}SITU}$ catalyst is also higher than that obtained with the *in situ* catalyst as shown in Figure 9. The same observation is also made at higher temperatures and under increasing concentrations of ethylbenzene [EB] in the reaction stream. The second most significant aspect of $\gamma$-Al$_2$O$_3$@C$_{EX\text{-}SITU}$ catalyst is that DDH activity remains extremely stable as a function of catalyst time-on-stream and no catalyst deactivation is observed after 200 h testing (Figure 9). The selectivity improves slightly when the EB concentration is increased, which can be explained by the existence of competitive adsorption phenomena between EB and ST formed at lower EB concentrations.

The porosity of the "ex-situ" treated $\gamma$-Al$_2$O$_3$, including the ex-situ material after DDH was also investigated. Figure 11 shows the existence of a rational trend for the variation of SSA and pore size distribution. Elemental and TGA analyses are used to calculate the C-loading on the different materials.

As it can be seen for the $\gamma$-Al$_2$O$_3$@C$_{EX\text{-}SITU}$ sample, SSA (Figure 11 A) and pore size distribution (Figure 11 B) do not change significantly from pristine $\gamma$-Al$_2$O$_3$ (see Figure 6A). This is coherent with the moderate C-content obtained for the $\gamma$-Al$_2$O$_3$@C$_{EX\text{-}SITU}$ sample.

From elemental analyses of samples before and after DDH we can reasonably exclude the presence of sulfur coming from possible sulfonic groups as Bronsted acid sites at the $\gamma$-Al$_2$O$_3$ surface.

X-ray photoelectron spectroscopy (XPS) measurements were carried out in an ultrahigh vacuum (UHV) spectrometer equipped with a VSW Class WA hemispherical electron analyzer. A monochromated Al K$\alpha$ X-ray source (1486.6 eV) was used as incident radiation and XP spectra were recorded using a pass energy of 44 eV. Survey and high-resolution spectra were recorded in constant pass energy mode (90 and 44 eV, respectively). All samples were mounted on Cu tapes. The Binding Energy (BE) values were corrected relatively to the C1s peak component at 284.6 eV when necessary. In Figure 10A, the survey XPS spectra of freshly prepared $\gamma$-Al$_2$O$_3$@C$_{EX\text{-}SITU}$ and $\gamma$-Al$_2$O$_3$@C$_{EX\text{-}SITU}$ after DDH test are shown. In both samples the oxygen, carbon and aluminum peaks relative ratio changes significantly. High resolution XPS spectra of the C 1s and Al 2p peaks were also acquired for $\gamma$-Al$_2$O$_3$@C$_{EX\text{-}SITU}$ and $\gamma$-Al$_2$O$_3$@C$_{IN\text{-}SITU}$ after DDH(Figure 10 B and C). If we compare the Al 2p peaks of the two "used" samples, they are identical. The C 1s main peak of $\gamma$-Al$_2$O$_3$@C$_{IN\text{-}SITU}$ and $\gamma$-Al$_2$O$_3$@C$_{EX\text{-}SITU}$ appear at ca. 284.4 $\pm$ 0.1 (sp$^2$ C). These peaks were analyzed in 5 components see Table 1 for the attribution of the peaks. From Table 1 it is evident that the relative sp$^2$/sp$^3$ ratio does not change (within the experimental error) while more oxidized carbon species exist in the $\gamma$-Al$_2$O$_3$@C$_{IN\text{-}SITU}$ sample. According to the XPS observation no sulfur or additional elements were detected and these results are in agreement with those obtained from the elemental analysis.

Table 1. Surface atomic ratios of different carbon species

| BE | | $\gamma$-Al$_2$O$_3$@C$_{EX\text{-}SITU}$ | $\gamma$-Al$_2$O$_3$@C$_{IN\text{-}SITU}$ |
|---|---|---|---|
| 284.3 | sp$^2$ | 63.4 | 57.8 |
| 285.2 | sp$^3$ | 13.4 | 12.1 |
| 286 | COH | 9.7 | 12.6 |
| 287.3 | CO | 5.9 | 8.5 |
| 288.6 | COOH | 3.7 | 5.3 |
| 290.2 | $\pi$-$\pi$* | 3.9 | 3.7 |

A significant difference is also observed concerning the specific surface area of the ex-situ catalyst compared to the in-situ one (Figure 6 vs. Figure 11). Indeed, for the « in-situ » prepared catalyst ($\gamma$-Al$_2$O$_3$@C$_{IN\text{-}SITU}$), the specific surface area (SSA) may decrease - for example - from 249 m$^2$/g in the pristine $\gamma$-Al$_2$O$_3$ to about 41 m$^2$/g (Figure 6 A vs. 6 C) in the C-coated composite (used catalyst) (see Figure 3 B). A significant modification of the pore size distribution is observed between pristine $\gamma$-Al$_2$O$_3$ and its C-coated composite, the latter showing smaller mesopores within a narrower pore-size distribution. (Figure 6 B vs. 6 D). On the other hand, once the « ex-situ » sample is treated in catalysis ($\gamma$-Al$_2$O$_3$@C$_{EX\text{-}SITU}$ in DDH), BET may vary - for example from 267 m$^2$/g to 184 m$^2$/g (Figure 11 A vs. 11 C) and pore size distribution is substantially confirmed or only slightly modified (Figure 11 B vs. 11D).

Elemental analysis of $\gamma$-Al$_2$O$_3$@C$_{EX\text{-}SITU}$ sample after DDH has shown a relatively high C- content: $\gamma$-Al$_2$O$_3$@C$_{EX\text{-}SITU}$ after DDH: C = 19.67 %, H = 0.9 %

Variable temperature FT-IR (VT FT-IR) on $\gamma$-Al$_2$O$_3$. Two samples have been analyzed in terms of Lewis and Bronsted acid groups content. Two samples of $\gamma$-Al$_2$O$_3$ have been treated at 150°C and 550°C, respectively, before proceeding with the titration of their acid functionalities with vapors of pyridine. Figures 12 A and B outlines the FT-IR spectra obtained for the two $\gamma$-Al$_2$O$_3$ samples (pre-heated at 150 °C and 550 °C, respectively). After treatment with pyridine vapors the distinctive band associated to the interaction between the base and the Lewis acid sites (**LAS**) appears oversaturated, at least for temperatures below the pyridine boiling point. The band at 1450 cm$^{-1}$ is oversaturated at 50 °C due to the presence of an excess of adsorbed pyridine groups. After this initial temperature, it is possible to verify the effective and quantitative nature of the acid groups available in the material. The absence of Brønsted acid sites mainly due to hydroxyl groups or sulfonic acid groups (Brønsted surface acid sites, **BAS**) is unambiguously demonstrated by the absence of any significant band around 1540 cm$^{-1}$. This result is comparable for both samples. While thermal treatment at 150 °C of $\gamma$-Al$_2$O$_3$ is expected to simply remove adsorbed water from the material surface and pores, the treatment at temperatures higher than 450 °C is expected to completely remove (if present) hydroxyl groups responsible of the Bronsted character of the material.

The main conclusion is that neither the sample treated at 150 °C nor the one treated at 550 °C present an appreciable amount of Bronsted acid groups. It can be concluded that these $\gamma$-Al$_2$O$_3$ samples are provided as already thermally treated materials. Therefore, only Lewis acid sites are available at the materials outer surfaces. This is in line with the other characterizations for this $\gamma$-Al$_2$O$_3$ samples. The presence of Lewis surface acid sites (**LAS**) is clearly demonstrated by the appearance of a distinctive band at 1448 cm$^{-1}$. From the integration of the IR band of the curve recorded at 150°C it is also possible to provide a quantitative analysis of the Lewis acid sites in the sample under measurement.

The following Table provides the exact amount of **LAS** on both samples. As it can be inferred from these data, both samples present comparable quantity of **LAS** (62 vs 55 $\mu$eq g$^{-1}$).

Table 2. Lewis acid sites (**LAS**) on $\gamma$-Al$_2$O$_3$ pre-treated at 150 °C and 550 °C determined by integrating the bands at 1448 cm$^{-1}$ of the spectra collected at 150 °C.

| T of pre-treatment (°C) | LAS ($\mu$eq g$^{-1}$) |
|---|---|
| 150 | **62** |
| 550 | **55** |

VT FT-IR on $\alpha$-Al$_2$O$_3$ shows that all hydroxyl groups are definitively removed from the material outer surface at 450 °C. Figure 13 A outlines the IR spectra using pristine $\alpha$-Al$_2$O$_3$ as background (to be subtracted). In the present case, titration with Pyridine vapors has revealed that neither Lewis acid sites nor Bronsted acid sites are detectable on the sample after treatment at high temperature (550 °C). This is reasonably due to the significantly low SSA of this sample (10 m$^2$/g for $\alpha$-Al$_2$O$_3$ vs. 249 m$^2$/g for $\gamma$-Al$_2$O$_3$). Figure 13 B and C refer to SSA and pore size distribution of $\alpha$-Al$_2$O$_3$.

On the most representative support ($\gamma$-Al$_2$O$_3$) we have also run VT XPRD measurements in order to verify the alteration of the crystallinity in the material upon heating. As it can be seen from Figure 14, no significant alteration of the original pattern is observed till 600 °C. It can be concluded, that no appreciable morphological changes take place on the $\gamma$-Al$_2$O$_3$ upon heating but only a modification of its chemical composition can be assumed. This is finally confirmed by BET and BJH (pore size distribution) analysis (see figures 15 A and B).

Figure 15 A and B reveal that only a negligible modification of SSA and pore size distribution take place on $\gamma$-Al$_2$O$_3$ after heating the sample at 550 °C under inert atmosphere (2h, Ar) (see Figures 6 A and B vs. Figure 15 A and B).

Additional data on the nature of the C-deposit can also be achieved from the temperature-programmed oxidation (TPO) analysis of the $\gamma$-Al$_2$O$_3$ sample after DDH ($\gamma$-Al$_2$O$_3$@C$_{INSITU}$). As Figure 16 shows, TPO does not show any high-temperature (HT) carbon deposit but only a low-temperature (LT) carbon coke is formed. Different combustion products as measured by the quadrupole at the reactor outlet, have also been reported for the sake of completeness. TGA analyses conducted up to 1100°C on air on $\gamma$-Al$_2$O$_3$ and $\gamma$-Al$_2$O$_3$@C$_{EXSITU}$ after DDH (Figures 3B and 8B) do not show any other (high temperature) C-deposit.

The catalyst $\gamma$-Al$_2$O$_3$@C$_{EX-SITU(MC)}$ (where the MC$_{EX-SITU}$ coating is a mesoporous C-layer obtained from impregnation of $\gamma$-Al$_2$O$_3$ in an aqueous mixture of dextrose and citric acid followed by a thermal treatment under nitrogen atmosphere at 600°C) was also investigated in the steam-free DDH process. The $\gamma$-Al$_2$O$_3$@C$_{EX-SITU(MC)}$ offers high and superior catalytic performance compared to $\gamma$-Al$_2$O$_3$@C$_{EX-SITU}$ at T$_{reaction}$= 550°C, however becomes less active under harsher conditions i.e. 600°C and 5-10 vol.% EB (Figure 17).

The benchmarking in terms of conversion and selectivity of pristine and *ex-situ* carbon coated $\gamma$-Al$_2$O$_3$ and SiC support is highlighted on Figure 18. The results show again the superior activity of the $\gamma$-Al$_2$O$_3$@C systems compare to SiC and SiC@MC catalysts.

The effect of nitrogen doping was also investigated. The *ex-situ* coated nitrogen doped carbon catalysts are prepared by impregnation of a $\gamma$-Al$_2$O$_3$ support (5g) with a homogeneous aqueous solution made of glucose (2g), citric acid (2g) and (3g) ammonium carbonate, followed by two successive thermal treatments: the first one at low temperature (130 °C in air) where the impregnating solution is dried on the support thus forming a tiny pre-catalytic layer, and the second one at 600 °C (heating rate of 10 °C/min) under inert atmosphere allowing the formation of the catalytically active C-layer (coating). The catalytic performance of the $\gamma$-Al$_2$O$_3$@NMC$_{EX-SITU}$ is almost similar to that recorded with the undoped $\gamma$-Al$_2$O$_3$@MC$_{EX-SITU}$ sample (Figure 19).

**Example 8: Comparison of metal-free catalysts with the benchmark industrial Fe-K / Al catalyst**

The results obtained by the inventors on the various catalysts without metals are compared with those obtained using the industrial catalyst Fe-K/Al under identical conditions. Results are outlined on Figure 20. All tests are operated at 600°C.

For a low concentration in EB, i.e. [EB] = 2.8 vol.%, the commercial Fe-K catalyst shows an ethylbenzene conversion ($C_{EB}$) of about 25% with a styrene selectivity ($S_{ST}$) of 90% while the $\gamma$-Al$_2$O$_3$@C$_{IN-SITU}$ and $\gamma$-Al$_2$O$_3$@C$_{EX-SITU}$ catalysts display a $C_{EB}$ of 62% and 75% with a $S_{ST}$ of 98% and 97%, respectively. A similar trend was observed at higher EB concentrations, i.e. [EB] = 5 and 10 vol.%. The catalytic performance of the $\gamma$-Al$_2$O$_3$@C$_{IN-SITU}$ and $\gamma$-Al$_2$O$_3$@C$_{EX-SITU}$ catalysts is about > 2 times higher than those obtained with the K-promoted iron industrial catalyst (noted K-Fe in Figure 20) in the absence of steam.

In conclusion, we can claim that the carbon layer, formed directly during the test of DDH or preliminarily deposited by thermal decomposition of a hydrocarbon at the surface of a $\gamma$-Al$_2$O$_3$-based support, acts as a highly active and selective metal-free catalyst for the DDH reaction of EB to ST, in the absence of water vapor (steam) and under reaction conditions similar to those commonly applied at industrial level. The results obtained also clearly indicate that this "active" carbonaceous phase can be formed only on $\gamma$-Al$_2$O$_3$ as support whereas other supports such as silica, silicon carbide or $\alpha$-Al$_2$O$_3$ (the latter featured by a lower specific surface area compared to its $\gamma$-Al$_2$O$_3$ counterpart) generate inactive catalytic composites. Evidences, support the idea that the formation of an "active" carbonaceous phase is likely promoted by the presence of Lewis acid groups available at the surface of the support.

**Example 9: Direct dehydrogenation of EB to ST using an *ex-situ* carbon catalyst supported on $\gamma$-Al$_2$O$_3$ under steam conditions**

The direct dehydrogenation reaction of EB to ST with $\gamma$-Al$_2$O$_3$@C$_{EX-SITU}$ as catalyst in the presence of various amount of steam i.e. 2.8, 5 and 10 vol.% is investigated in this last section. Figure 21A shows marked instability of the catalyst performance ($\gamma$-Al$_2$O$_3$@C$_{EX-SITU}$ - previously activated under steam-free feed at 600°C) under a steam feed of 2.8, 5 and 10 vol.%. A higher catalyst stability was observed when the process was conducted using 10 vol.% of steam, however a drastic decrease of ethylbenzene conversion ($C_{EB}$) from 66% to 50% after a dozen of hours of reaction takes place. For better understanding the steam effect, the reaction was carried out by activating the $\gamma$-Al$_2$O$_3$@C$_{EX-SITU}$ directly with the reactant feed containing steam under conditions where the catalyst is stable i.e. 10 vol. %. Results show a drastic decrease of the catalytic performance which increases after removal of steam in the reactant feed (Figure 21 B). According to the observed results it can be stated that the presence of steam during the activation period have a significant influence on the DDH performance of the catalyst. Anyway, the full DDH performance is almost recovered after returning to the steam-free conditions as evidenced in Figure 21B, even for the catalyst activated directly under a high concentration of steam.

**List of references**

**1.** R. A. Meyer. Handbook of Petrochemicals Production processes. New-York NY, McGraw-Hill, 11.3-11.34 (2005).

**2.** T. G. Alkhazov, A. E. Lisovskii. Role of condensation products in oxidative dehydrogenation process of ethylbenzene on alumina-oxide catalyst. Kinet. Catal. 17, 375-379, 1976.

**3.** G. E. Vrieland, P. G. Menon. Nature of the catalytically active carbonaceous sites for the oxydehydrogenation of ethylbenzene to styrene - A brief review. Appl. Catal. 77, 1-8, 1991.

**4.** C. Nederlof, F. Kapteijn, M. Makkee. Catalysed ethylbenzene dehydrogenation in CO2 or N2 - Carbon deposits as the active phase. Appl. Catal. A: Gen. 417, 163-173, 2012.

**5.** N. Keller, N. I. Maksimova, V. V. Roddatis, M. Schur, G. Mestl, Y. V. Butenko, V. L. Kuznetsov, R. Schlogl. The catalytic use of onion-like carbon materials for styrene synthesis by oxidative dehydrogenation of ethylbenzene. Angew. Chem. Int. Ed. 41, 1885-1888, 2002.

**6.** M. F. R. Pereira, J. L. Figueiredo, J. J. M. Orfao, P. Serp, P. Kalck, Y. Kihn. Catalytic activity of carbon nanotubes in the oxidative dehydrogenation of ethylbenzene. Carbon 42, 2807-2813, 2004.

**7.** R. Fiedorow, W. Przystajko, M. Sopa, I. G. Dalla Lana. The nature and catalytic influence of coke formed on alumina: oxidative dehydrogenation of ethylbenzene. J. Catal. 68, 33-41, 1981.

**8.** J. Zhang, D. S. Su. R. Blume, R. Schlögl, R. Wang, X. Yang, A. Gajovic. Surface Chemistry and Catalytic Reactivity of a nanodiamond in the Steam-Free dehydrogenation of Ethylbenzene. Angew. Chem. Int. Ed. 49, 8640-8644, 2010.

**9.** H. Ba, L. Truong-Phuoc, Y. Liu, C. Duong-Viet, J. M. Nhut, L. Nguyen-Dinh, P. Granger, C. Pham-Huu. Hierarchical carbon nanofibers/graphene composite containing nanodiamonds as metal-free catalyst for direct dehydrogenation of ethylbenzene. Carbon 96, 1060-1069, 2016.

**10.** H. Ba, Y. Liu, X. Mu, J. M. Nhut, W. H. Doh, P. Granger, C. Pham-Huu. Nanodiamonds decorated silicon carbide foam as metal-free catalyst for steam-free ethylbenzene dehydrogenation. Appl. Catal. A: Gen. 499, 217-226, 2015.

**11.** V. Zarubina, C. Nederlof, B. Van der Linden, F. Kapteijn, H. J. Heeres, M. Makkee, I. Melian-Cabrera. Making coke a more efficient catalyst in the oxidative dehydrogenation of ethylbenzene using wide-pore transitional alumina. J. Mol. Catal. A: Chem. 381, 179-187, 2014.

**12.** T. Wang, S. Chong, T. Wang, H. Lu, M. Ji. The physicochemical properties and catalytic performance of carbon-covered alumina for oxidative dehydrogenation of ethylbenzene with CO2. Appl. Surf. Sci. 427, 1011-1018, 2018.

**13.** Y. Liu, H. Ba, J. Luo, J. M. Nhut, D. S. Su, C. Pham-Huu. Structure-performance relationship of nanodiamonds@nitrogen-doped mesoporous carbon in the direct dehydrogenation of ethylbenzene. Catal. Today 301, 38-47, 2018.

## Claims

**1.** Process for the direct dehydrogenation of a hydrocarbon, comprising the step of contacting said hydrocarbon with a catalyst at a temperature comprised between 400°C and 700 °C, wherein said catalyst consists of a carbon active phase that is coated on $\gamma$-Al$_2$O$_3$ support, said $\gamma$-Al$_2$O$_3$ support being a host matrix for said carbon active phase.

**2.** Process according to claim 1, wherein said $\gamma$-Al$_2$O$_3$ support is (i) a macroscopic support for hosting the carbon active phase or (ii) a coating layer on other insulator or thermal conductive supports for hosting the carbon active phase.

**3.** Process according to claim 2, wherein said thermal conductive support comprises carbon or silicon carbide.

**4.** Process according to claim 2, wherein said support for hosting a $\gamma$-Al$_2$O$_3$ coating is chosen among silicon carbide, $\alpha$-Al$_2$O$_3$ or other alumina phases, TiO$_2$, carbon and stainless steel.

**5.** Process according to anyone of the preceding claims, wherein said $\gamma$-Al$_2$O$_3$ is deposited onto the support through impregnation of a colloidal $\gamma$-Al$_2$O$_3$ solution (sol) or a suspension of $\gamma$-Al$_2$O$_3$ powder or through ball milling process.

**6.** Process according to anyone of the preceding claims, wherein said catalyst is formed *in-situ* during said direct dehydrogenation reaction by passing over said $\gamma$-Al$_2$O$_3$ support a hydrocarbon gas diluted in an inert gas stream.

**7.** Process according to anyone of the preceding claims, wherein said catalyst is formed *ex-situ* before the direct dehydrogenation reaction by submitting the $\gamma$-Al$_2$O$_3$ support to a stream of a gaseous hydrocarbon, diluted in an inert gas, said stream having a temperature comprised between 500°C and 700°C, for a duration between 2 to 10 h.

**8.** Process according to anyone of the preceding claims, wherein said direct dehydrogenation reaction is realized for a period of at least 600 hours.

**9.** Process according to anyone of the preceding claims, wherein said direct dehydrogenation reaction is carried out substantially in the absence of oxygen.

**10.** Process according to anyone of the preceding claims, wherein said direct dehydrogenation reaction is carried out in the absence of additional steam in the reactant feed.

11. Process according to anyone of the preceding claims, wherein said hydrocarbon is selected in the group consisting of ethylbenzene, ethane, propane, iso-butane, normal butane and cyclic hydrocarbons.

12. Process according to claim 11, wherein said hydrocarbon is ethylbenzene.

13. Process for the preparation of a composite comprising a carbon active phase coating on a $\gamma$-$Al_2O_3$ support, said $\gamma$-$Al_2O_3$ support being a host matrix for said carbon active phase, said process comprising the step of treating a support consisting of $\gamma$-$Al_2O_3$ at a temperature comprised of from 550°C to 650°, under an ethane stream for a period of at least 1.5 hours or under a stream of ethylbenzene, ethane or ethylbenzene diluted in an inert gas stream.

14. Process according to claim 13, wherein said inert gas is selected among helium, nitrogen and argon, or a combination thereof.

15. A composite comprising a support consisting of $\gamma$-$Al_2O_3$ and a carbon active phase, wherein said $\gamma$-$Al_2O_3$ support is a host matrix for said carbon active phase, obtainable by a process as defined in claim 13 or 14 or during a process as defined in anyone of claims 1 to 12.

16. Use of a composite as defined in claim 15, for oxygen- and/or steam-free direct dehydrogenation of ethylbenzene to styrene.

Figure 1

Figure 2

Figure 3

Figure 4

Figure 5

Figure 6

Figure 7

Figure 8

Figure 9

Figure 10

Figure 11

Figure 12

Figure 13

Figure 14

Figure 15

Figure 16

Figure 17

Figure 18

Figure 19

Figure 20

Figure 21

Europäisches
Patentamt

European
Patent Office

Office européen
des brevets

## EUROPEAN SEARCH REPORT

**Application Number**

EP 18 30 5336

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GB 1 156 532 A (MONSANTO CO [US]) 25 June 1969 (1969-06-25) * examples 1,6,7,9 * | 1-12,16 | INV. C07C5/333 C07C15/46 B01J21/04 |
| A,D | ZARUBINA V ET AL: "Making coke a more efficient catalyst in the oxidative dehydrogenation of ethylbenzene using wide-pore transitional aluminas", JOURNAL OF MOLECULAR CATALYSIS A: CHEMICAL, vol. 381, 28 October 2013 (2013-10-28), pages 179-187, XP028785782, ISSN: 1381-1169, DOI: 10.1016/J.MOLCATA.2013.10.010 * abstract * | 1-12,16 | B01J21/18 B01J37/02 B01J37/08 |
| A,D | NEDERLOF CHRISTIAN ET AL: "Catalysed ethylbenzene dehydrogenation in CO2or N2-Carbon deposits as the active phase", APPLIED CATALYSIS A: GENERAL, vol. 417, 31 December 2011 (2011-12-31), pages 163-173, XP028891399, ISSN: 0926-860X, DOI: 10.1016/J.APCATA.2011.12.037 * section 2.1 * | 1-12,16 | |
| | | | TECHNICAL FIELDS SEARCHED (IPC) |
| | | | C07C B01J |
| A | WO 2006/008049 A1 (NANOC SDN BHD; SCHLOEGL ROBERT [DE]; MESTL GERHARD [DE]) 26 January 2006 (2006-01-26) * last three lines of page 2; claim 1 * | 1-12,16 | |

~~The present search report has been drawn up for all claims~~

| Place of search | Date of completion of the search | Examiner |
|---|---|---|
| The Hague | 26 September 2018 | Matés Valdivielso, J |

EPO FORM 1503 03.82 (P04C01)

Europäisches Patentamt

European Patent Office

Office européen des brevets

Application Number

EP 18 30 5336

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-12(completely); 16(partially)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 18 30 5336

The Search Division considers that the present European patent application does not comply with the
requirements of unity of invention and relates to several inventions or groups of inventions, namely:

    1. claims: 1-12(completely); 16(partially)

        Process for the direct dehydrogenation of a hydrocarbon.
                        ---

    2. claims: 13-15(completely); 16(partially)

        Process for the preparation of a composite comprising a
        carbon active phase coating on a gamma-Al2O3 support.
                        ---

## ANNEX TO THE EUROPEAN SEARCH REPORT
## ON EUROPEAN PATENT APPLICATION NO.

EP 18 30 5336

This annex lists the patent family members relating to the patent documents cited in the above-mentioned European search report.
The members are as contained in the European Patent Office EDP file on
The European Patent Office is in no way liable for these particulars which are merely given for the purpose of information.

26-09-2018

| Patent document cited in search report | | Publication date | Patent family member(s) | | Publication date |
|---|---|---|---|---|---|
| GB 1156532 | A | 25-06-1969 | BE | 682863 A | 21-12-1966 |
| | | | DE | 1593042 A1 | 23-07-1970 |
| | | | GB | 1156532 A | 25-06-1969 |
| WO 2006008049 | A1 | 26-01-2006 | CN | 101014412 A | 08-08-2007 |
| | | | EP | 1773491 A1 | 18-04-2007 |
| | | | JP | 5377851 B2 | 25-12-2013 |
| | | | JP | 2008506514 A | 06-03-2008 |
| | | | US | 2008071124 A1 | 20-03-2008 |
| | | | WO | 2006008049 A1 | 26-01-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **R. A. MEYER.** Handbook of Petrochemicals Production processes. McGraw-Hill, 2005, 11.3-11.34
- **T. G. ALKHAZOV ; A. E. LISOVSKII.** Role of condensation products in the oxidative dehydrogenation process of ethylbenzene on alumina-oxide catalyst. *Kinet. Catal.,* 1976, vol. 17, 375-379
- **G. E. VRIELAND ; P. G. MENON.** Nature of the catalytically active carbonaceous sites for the oxydehydrogenation of ethylbenzene to styrene - A brief review. *Appl. Catal.,* 1991, vol. 77, 1-8
- **C. NEDERLOF ; F. KAPTEIJN ; M. MAKKEE.** Catalysed ethylbenzene dehydrogenation in CO2 or N2 - Carbon deposits as the active phase. *Appl. Catal. A: Gen.,* 2012, vol. 417, 163-173
- **N. KELLER ; N. I. MAKSIMOVA ; V. V. RODDATIS ; M. SCHUR ; G. MESTL ; Y. V. BUTENKO ; V. L. KUZNETSOV ; R. SCHLOGL.** The catalytic use of onion-like carbon materials for styrene synthesis by oxidative dehydrogenation of ethylbenzene. *Angew. Chem. Int. Ed.,* 2002, vol. 41, 1885-1888
- **M. F. R. PEREIRA ; J. L. FIGUEIREDO ; J. J. M. ORFAO ; P. SERP ; P. KALCK ; Y. KIHN.** Catalytic activity of carbon nanotubes in the oxidative dehydrogenation of ethylbenzene. *Carbon,* 2004, vol. 42, 2807-2813
- **VRIELAND ; MENON ; R. FIEDOROW ; W. PRZYSTAJKO ; M. SOPA ; I. G. DALLA LANA.** The nature and catalytic influence of coke formed on alumina: oxidative dehydrogenation of ethylbenzene. *J. Catal.,* 1981, vol. 68, 33-41
- **V. ZARUBINA ; C. NEDERLOF ; B. VAN DER LINDEN ; F. KAPTEIJN ; H. J. HEERES ; M. MAKKEE ; I. MELIAN-CABRERA.** Making coke a more efficient catalyst in the oxidative dehydrogenation of ethylbenzene using wide-pore transitional alumina. *J. Mol. Catal. A: Chem.,* 2014, vol. 381, 179-187
- **T. WANG ; S. CHONG ; T. WANG ; H. LU ; M. JI.** The physicochemical properties and catalytic performance of carbon-covered alumina for oxidative dehydrogenation of ethylbenzene with CO. *Appl. Surf. Sci.,* 2018, vol. 427, 1011-1018

- **J. ZHANG ; D. S. SU. R. BLUME ; R. SCHLÖGL ; R. WANG ; X. YANG.** A. Gajovic. Surface Chemistry and Catalytic Reactivity of a nanodiamond in the Steam-Free dehydrogenation of Ethylbenzene. *Angew. Chem. Int. Ed.,* 2010, vol. 49, 8640-8644
- **H. BA ; L. TRUONG-PHUOC ; Y. LIU ; C. DUONG-VIET ; J. M. NHUT ; L. NGUYEN-DINH ; P. GRANGER ; C. PHAM-HUU.** Hierarchical carbon nanofibers/graphene composite containing nanodiamonds as metal-free catalyst for direct dehydrogenation of ethylbenzene. *Carbon,* 2016, vol. 96, 1060-1069
- **H. BA ; Y. LIU ; X. MU ; J. M. NHUT ; W. H. DOH ; P. GRANGER ; C. PHAM-HUU.** Nanodiamonds decorated silicon carbide foam as metal-free catalyst for steam-free ethylbenzene dehydrogenation. *Appl. Catal. A: Gen.,* 2015, vol. 499, 217-226
- **Y. LIU ; H. BA ; J. LUO ; J. M. NHUT ; D. S. SU ; C. PHAM-HUU.** Structure-performance relationship of nanodiamonds@nitrogen-doped mesoporous carbon in the direct dehydrogenation of ethylbenzene. *Catal. Today,* 2018, vol. 301, 38-47
- **T. G. ALKHAZOV ; A. E. LISOVSKII.** Role of condensation products in oxidative dehydrogenation process of ethylbenzene on alumina-oxide catalyst. *Kinet. Catal.,* 1976, vol. 17, 375-379
- **R. FIEDOROW ; W. PRZYSTAJKO ; M. SOPA ; I. G. DALLA LANA.** The nature and catalytic influence of coke formed on alumina: oxidative dehydrogenation of ethylbenzene. *J. Catal.,* 1981, vol. 68, 33-41
- **J. ZHANG ; D. S. SU. R. BLUME ; R. SCHLÖGL ; R. WANG ; X. YANG ; A. GAJOVIC.** Surface Chemistry and Catalytic Reactivity of a nanodiamond in the Steam-Free dehydrogenation of Ethylbenzene. *Angew. Chem. Int. Ed.,* 2010, vol. 49, 8640-8644
- **T. WANG ; S. CHONG ; T. WANG ; H. LU ; M. JI.** The physicochemical properties and catalytic performance of carbon-covered alumina for oxidative dehydrogenation of ethylbenzene with CO. *Appl. Surf. Sci.,* 2018, vol. 427, 1011-1018